# EUROPEAN PATENT APPLICATION

(11) **EP 2 397 159 A2**
(43) Date of publication of application: **21.12.2011**
(21) Application number: 11180608.9
(22) Date of filing: 30.10.2009
(51) Int. Cl.: A61K 45/06, A61K 31/485, A61K 31/135, A61K 31/138, A61K 31/343, A61K 31/4525, A61P 25/04

(54) **Combination of morphinan compounds and antidepressant for the treatment of intractable and chronic pain**

(30) Priority: 30.10.2008 US 109833 P
(62) Divisional of application: 09744581.1
(71) Applicant: CONCERT PHARMACEUTICALS, INC., Lexington, MA 02421 (US)
(72) Inventor: Thomas, Amanda, Watertown, MA Massachusetts 02472 (US)
(74) Representative: Duncan, Garreth Andrew

(57) **Abstract**

Provided herein are compositions comprising a dextromethorphan analog according to Formula I or Formula II or a pharmaceutically acceptable salt of either of the foregoing and a co-agent, eg., an antidepressant such as a serotonin norepinephrine reuptake inhibitor; a serotonin noradrenaline dopamine reuptake inhibitor; a norepinephrine dopamine reuptake inhibitor; a monoamine oxidase inhibitor; a selective serotonin, reuptake inhibitor; and a tricyclic antidepressant or a pharmaceutically acceptable salt of any of the foregoing. The compositions are useful in the the treatment of pseudobulbar affect, neuropathic pain, neurodegenerative diseases, brain injuries, and the like.

## Description

### RELATED APPLICATION

This application claims priority to U.S. Provisional Application No. 61/109,833, filed October 30, 2008, which is incorporated by reference herein in its entirety.

### TECHNICAL FIELD

This disclosure relates to novel compositions and methods useful in the treatment of pseudobulbar affect, chronic or intractable pain, neurodegenerative diseases, and brain injuries.

### BACKGROUND

Dextromethorphan, also known by its chemical name (+)-3-methoxy-17-methyl-(9α,13α,14α)-morphinan, is currently one of the most widely used antitussives.

In addition to the physiological activity noted above, dextromethorphan is also an agonist of the σ2 receptor, an N-methyl-D-aspartate (NMDA) antagonist, and an α3β4 nicotinic receptor antagonist. Dextromethorphan inhibits neurotransmitters, such as glutamate, from activating receptors in the brain. Uptake of dopamine and serotonin are also inhibited.

Dextromethorphan is approved for use in over the counter cough suppressant products. It is currently in Phase I clinical trials for treating subjects with voice spasms, and Phase III clinical studies for treating Rett Syndrome (http://www.clinicaltrials.gov). Dextromethorphan is being studied with other drugs in a Phase II clinical trial characterizing pain processing mechanisms in subjects with irritable bowel syndrome (http://www.clinicaltrials.gov/). Dextromethorphan is also in Phase I clinical trials for treating hyperalgesia in methadone-maintained subjects (http://www.clinicaltrials.gov/).

Dextromethorphan when administered alone has also shown limited efficacy in the treatment of other diseases and conditions, including involuntary emotional expression disorder ("IEED") or pseudobulbar affect ("PBA"), neurodegenerative diseases, neuropathic pain, and brain injuries.

Although dextromethorphan has shown therapeutic effect in the above mentioned conditions and disorders, its rapid first-pass metabolism remains a major obstacle in the development of effective treatments. Dextromethorphan is metabolized in the liver. Degradation begins with O- and N-demethylation to form primary metabolites dextrorphan and 3-methoxy-morphinan, both of which are further N- and O- demethylated respectively to 3-hydroxy-morphinan. These three metabolites are believed to be therapeutically active. A major metabolic catalyst is the cytochrome P450 enzyme 2D6 (CYP2D6), which is responsible for the O-demethylation reactions of dextromethorphan and 3-methoxymorphinan. N-demethylation of dextromethorphan and dextrorphan are catalyzed by enzymes in the related CYP3A family. Conjugates of dextrorphan and 3-hydroxymorphinan can be detected in human plasma and urine within hours of its ingestion.

A combination of dextromethorphan hydrobromide and quinidine sulfate is currently in Phase III clinical trials for the treatment of PBA in patients suffering from Alzheimer's disease, stroke, Parkinson's disease and traumatic brain injury. (http://www.clinicaltrials.gov). The co-administration of quinidine (a potent inhibitor of the cytochrome P450 enzyme 2D6) increases both the blood level and the duration of action of dextromethorphan.

PBA is a neurological disorder characterized by inappropriate and uncontrollable outbursts of crying, laughing, or other emotional displays, often times with no relevant trigger or disproportionate with the actual mood of the person. Although rarely life threatening, PBA can significantly impact a person's professional and social life. PBA is commonly associated with certain neurological disorders such as traumatic brain injury, multiple sclerosis, amyotrophic lateral sclerosis, Alzheimer's disease, Parkinson disease, traumatic brain injury, progressive supranuclear palsy, multiple systems atrophy, normal pressure hydrocephalus, olivopontine cerebellar atrophy, brain tumors, Wilson's disease, and stroke. Over one million people in the United States suffer from PBA.

At the present time there are no treatments specifically approved by the Food and Drug Administration for the treatment of PBA. First line treatment for PBA is limited to the off-label use of antidepressants. Studies have demonstrated the therapeutic effect of tricylic antidepressants and selective serotonin reuptake inhibitors in the treatment of PBA. These agents are believed to have PBA-specific therapeutic effects independent of their antidepressant action. Antidepressants that have shown a therapeutic effect include amitriptyline, nortriptyline, citalopram, fluoxetine, paroxetine, and sertraline.

Although treatments for neurological diseases and conditions are known, there still exists a need to develop more efficacious treatments. The present disclosure fulfills this need and has other related advantages.

### SUMMARY

In one embodiment, provided is a method of treating pseudobulbar affect in a subject in need thereof, comprising the step of administering to the subject a therapeutically effective amount of a co-agent selected from the grouping consisting of a serotonin norepinephrine reuptake inhibitor; a serotonin noradrenaline dopamine reuptake inhibitor; a norepinephrine dopamine reuptake inhibitor; a monoamine oxidase inhibitor; a selective serotonin reuptake inhibitor; and a tricyclic antidepressant; or pharmaceutically acceptable salts thereof, and a therapeutically effective amount of a compound selected from the group consisting of a compound of Formula I: 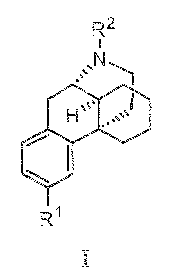 or a pharmaceutically acceptable salt thereof, wherein:
R¹ is selected from -O-(C₂-C₄)alkyl and -(C₁-C₄)alkyl; and
R² is-CH₃;
and a compound of Formula II: 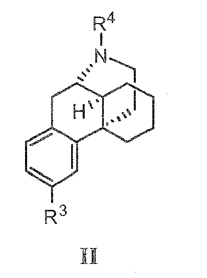 or a pharmaceutically acceptable salt thereof, wherein:
R³ is -OCH₃; and
R⁴ is -CH₃.

In certain embodiments the compound is a compound of Formula I. In certain embodiments, R¹ is -O-CH₂CH₃, -O-CH(CH₃)₂, -CH₃, -CH₂CH₃, or -CH₂CH(CH₃)₂.

In another aspect, the co-agent is an inhibitor of a cytochrome p450 2D6 enzyme.

In another aspect, the co-agent is selected from the group consisting of a serotonin norepinephrine reuptake inhibitor; a serotonin noradrenaline dopamine reuptake inhibitor; a norepinephrine dopamine reuptake inhibitor; norfluoxetine, citalopram, dapoxetine, escitalopram, fluvoxamine, paroxetine, sertraline, butriptyline, amoxapine, amitriptyline, clomipramine, desipramine, dosulepin, doxepin, imipramine, dibenzepin, iprindole, lofepramine, opipramol, protriptyline, and trimipramine, or pharmaceutically acceptable salts thereof.

In another aspect, the co-agent is a serotonin norepinephrine reuptake inhibitor selected from the group consisting of venlafaxine, desvenlafaxine, sibutramine, nefazodone, milnacipran, duloxetine, and bicifadine, or pharmaceutically acceptable salts thereof.

In certain instances, the co-agent is a serotonin norepinephrine reuptake inhibitor selected from the group consisting of venlafaxine, desvenlafaxine, nefazodone, and duloxetine, or pharmaceutically acceptable salts thereof.

In certain instances, the co-agent is a serotonin noradrenaline dopamine reuptake inhibitor selected from the group consisting of tesofensine and brasofensine, or pharmaceutically acceptable salts thereof.

In certain instances, the co-agent is a monoamine oxidase inhibitor selected from the group consisting of isocarboxazid, moclobemide, phenelzine, tranylcypromine, selegiline, rasagiline, nialamide, iproniazid, iproclozide, and toloxatone, or pharmaceutically acceptable salts thereof.

In certain instances, the co-agent is a tricyclic antidepressant selected from the group consisting of butriptyline, amoxapine, amitriptyline, nortriptyline, clomipramine, desipramine, dosulepin, doxepin, imipramine, dibenzepin, iprindole, lofepramine, opipramol, protriptyline, and trimipramine, or pharmaceutically acceptable salts thereof.

In certain instances, the co-agent is a tricyclic antidepressant selected from the group consisting of amitriptyline, clomipramine, desipramine, doxepin, and imipramine, or pharmaceutically acceptable salts thereof.

In certain instances, the co-agent is a selective serotonin reuptake inhibitor selected from the group consisting of fluoxetine, norfluoxetine, citalopram, dapoxetine, escitalopram, fluvoxamine, paroxetine, and sertraline, or pharmaceutically acceptable salts thereof.

In certain instances, the co-agent is a selective serotonin reuptake inhibitor selected from the group consisting of citalopram, norfluoxetine, dapoxetine, escitalopram, fluvoxamine, paroxetine, and sertraline, or pharmaceutically acceptable salts thereof.

In certain instances, the co-agent is paroxetine, or a pharmaceutically acceptable salt thereof.

In another embodiment, provided is a method of treating pseudobulbar affect in a subject in need thereof, comprising the step of administering to the subject a therapeutically effective amount of a co-agent selected from the grouping consisting of citalopram, fluvoxamine, norfluoxetine, fluoxetine, paroxetine, sertraline, venlafaxine, desvenlafaxine, nefazodone, duloxetine, bupropion, moclobemide, amitriptyline, clomipramine, desipramine, doxepin, imipramine and nortriptyline, or pharmaceutically acceptable salts thereof, and a therapeutically effective amount of a compound selected from the group consisting of: 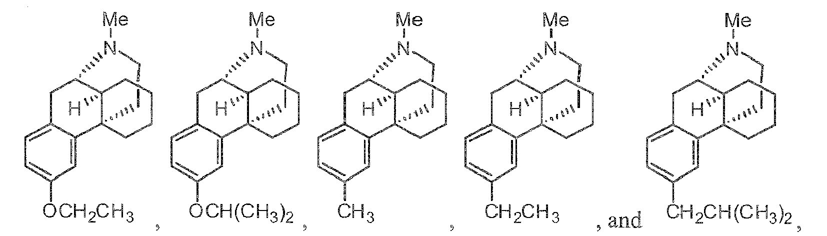 or pharmaceutically acceptable salts thereof.

In another embodiment, provided is a method of treating pseudobulbar affect in a subject in need thereof, comprising the step of administering to the subject a therapeutically effective amount of a co-agent selected from the grouping consisting of citatopram, fluvoxamine, norfluoxetine, fluoxetine, paroxetine, sertraline, venlafaxine, desvenlafaxine, nefazodone, duloxetine, bupropion, moclobemide, amitriptyline, clomipramine, desipramine, doxepin, imipramine and nortriptyline, or pharmaceutically acceptable salts thereof, and a therapeutically effective amount of a compound with formula: 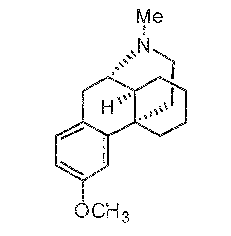 or a pharmaceutically acceptable salt thereof.

In certain instances, the co-agent is citalopram, fluvoxamine, paroxetine, sertraline, venlafaxine, desvenlafaxine, nefazodone, duloxetine, bupropion, moclobemide, clomipramine, desipramine, doxepin, or imipramine, or pharmaceutically acceptable salts thereof.

In certain instances, the co-agent is paroxetine, or a pharmaceutically acceptable salt thereof.

Also provided is a method of treating chronic or intractable pain in a subject in need thereof, comprising the step of administering to the subject a therapeutically effective amount of a co-agent selected from the grouping consisting of a serotonin norepinephrine reuptake inhibitor; a serotonin noradrenaline dopamine reuptake inhibitor; a norepinephrine dopamine reuptake inhibitor; a monoamine oxidase inhibitor; a tricyclic antidepressant; and a selective serotonin reuptake inhibitor; or pharmaceutically acceptable salts thereof; and a a therapeutically effective amount of a compound selected from the group consisting of a compound of Formula **I**: 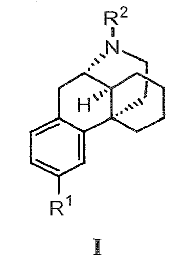 or a pharmaceutically acceptable salt thereof, wherein:
R¹ is selected from -O-(C₂-C₄)alkyl and -(C₁-C₄)alkyl; and
R² is -CH₃;
and a compound of Formula **II**: 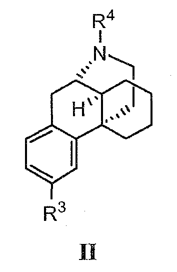 or a pharmaceutically acceptable salt thereof, wherein:
R³ is -OCH₃; and
R⁴ is -CH₃.

In certain embodiments the compound is a compound of Formula I. In certain embodiments, R¹ is -O-CH₂CH₃, -O-CH(CH₃)₂, -CH₃, -CH₂CH₃, or -CH₂CH(CH₃)₂.

In certain instances, the co-agent is an inhibitor of a cytochrome p450 2D6 enzyme.

In certain instances, the co-agent is selected from the group consisting of a serotonin norepinephrine reuptake inhibitor; a serotonin noradrenaline dopamine reuptake inhibitor; a norepinephrine dopamine reuptake inhibitor; norfluoxetine, citalopram, dapoxetine, escitalopram, fluvoxamine, paroxetine, sertraline, butriptyline, amoxapine, amitriptyline, clomipramine, desipramine, dosulepin, doxepin, imipramine, dibenzepin, iprindole, lofepramine, opipramol, protriptyline, and trimipramine, or pharmaceutically acceptable salts thereof.

In certain instances, the co-agent is a serotonin norepinephrine reuptake inhibitor selected from the group consisting of venlafaxine, desvenlafaxine, sibutramine, nefazodone, milnacipran, duloxetine, and bicifadine, or pharmaceutically acceptable salts thereof.

In certain instances, the co-agent is a serotonin norepinephrine reuptake inhibitor selected from the group consisting of venlafaxine, desvenlafaxine, nefazodone, and duloxetine, or pharmaceutically acceptable salts thereof.

In certain instances, the co-agent is a serotonin noradrenaline dopamine reuptake inhibitor selected from the group consisting of tesofensine and brasofensine, or pharmaceutically acceptable salts thereof.

In certain instances, the co-agent is a monoamine oxidase inhibitor selected from the group consisting of isocarboxazid, moclobemide, phenelzine, tranylcypromine, selegiline, rasagiline, nialamide, iproniazid, iproclozide, and toloxatone, or pharmaceutically acceptable salts thereof.

In certain instances, the co-agent is a tricyclic antidepressant selected from the group consisting of butriptyline, amoxapine, amitriptyline, nortriptyline, clomipramine, desipramine, dosulepin, doxepin, imipramine, dibenzepin, iprindole, lofepramine, opipramol, protriptyline, and trimipramine, or pharmaceutically acceptable salts thereof.

In certain instances, the co-agent is a tricyclic antidepressant selected from the group consisting of amitriptyline, clomipramine, desipramine, doxepin, and imipramine, or pharmaceutically acceptable salts thereof.

In certain instances, the co-agent is a selective serotonin reuptake inhibitor selected from the group consisting of fluoxetine, norfluoxetine, citalopram, dapoxetine, escitalopram, fluvoxamine, paroxetine, and sertraline, or pharmaceutically acceptable salts thereof.

In certain instances, the co-agent is selective serotonin reuptake inhibitor selected from the group consisting of citalopram, norfluoxetine, dapoxetine, escitalopram, fluvoxamine, paroxetine, and sertraline, or pharmaceutically acceptable salts thereof.

In certain instances, the co-agent is paroxetine, or a pharmaceutically acceptable salt thereof.

In another embodiment, provided is a method of treating chronic or intractable pain in a subject in need thereof, comprising the step of administering to the subject a therapeutically effective amount of a co-agent selected from the grouping consisting of citalopram, fluvoxamine, norfluoxetine, fluoxetine, paroxetine, sertraline, venlafaxine, desvenlafaxine, nefazodone, duloxetine, bupropion, moclobemide, amitriptyline, clomipramine, desipramine, doxepin, imipramine and nortriptyline, or pharmaceutically acceptable salts thereof, and a therapeutically effective amount of a compound selected from the group consisting of: 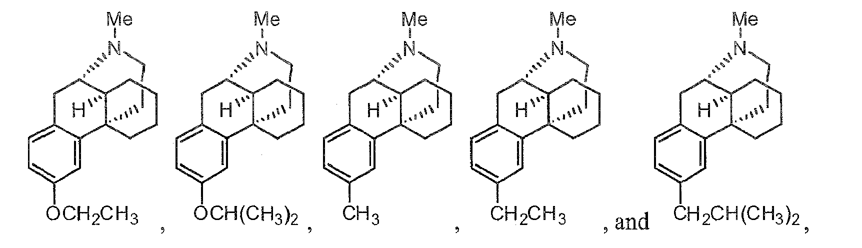 or pharmaceutically acceptable salts thereof.

In another embodiment, provided is a method of treating chronic or intractable pain in a subject in need thereof, comprising the step of administering to the subject a therapeutically effective amount of a co-agent selected from the grouping consisting of citalopram, fluvoxamine, norfluoxetine, fluoxetine, paroxetine, sertraline, venlafaxine, desvenlafaxine, nefazodone, duloxetine, bupropion, moclobemide, amitriptyline, clomipramine, desipramine, doxepin, imipramine and nortriptyline, or pharmaceutically acceptable salts thereof, and a therapeutically effective amount of a compound with formula: 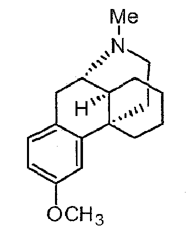 or a pharmaceutically acceptable salt thereof.

In certain instances, the co-agent is citalopram, fluvoxamine, paroxetine, sertraline, venlafaxine, desvenlafaxine, nefazodone, duloxetine, bupropion, moclobemide, clomipramine, desipramine, doxepin, or imipramine, or pharmaceutically acceptable salts thereof.

In certain instances, the co-agent is paroxetine, or a pharmaceutically acceptable salt thereof.

Another embodiment relates to any of the aforementioned methods for treating chronic or intractable pain, where the chronic or intractable pain is a neuropathic pain.

Another embodiment relates to any of the aforementioned methods for treating chronic or intractable pain, where the chronic or intractable pain is diabetic neuropathic pain.

In another embodiment, provided is a method of treating a neurological disorder selected from the group consisting of amyotrophic lateral sclerosis, multiple sclerosis, Parkinson's disease, Alzheimer's disease, and Huntington's disease, comprising the step of administering to the subject a therapeutically effective amount of a co-agent selected from the grouping consisting of a serotonin norepinephrine reuptake inhibitor; a serotonin noradrenaline dopamine reuptake inhibitor; a norepinephrine dopamine reuptake inhibitor; a monoamine oxidase inhibitor; a tricyclic antidepressant; and a selective serotonin reuptake inhibitor; or pharmaceutically acceptable salts thereof; and a a therapeutically effective amount of a compound selected from the group consisting of a compound of Formula **I**: 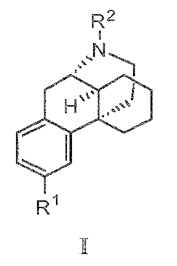 or a pharmaceutically acceptable salt thereof, wherein:
R¹ is selected from -O-(C₂-C₄)alkyl and -(C₁-C₄)alkyl; and
R² is -CH₃;
and a compound of Formula **II**: 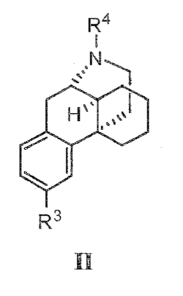 or a pharmaceutically acceptable salt thereof, wherein:
R³ is -OCH₃; and
R⁴ is -CH₃.

In certain embodiments the compound is a compound of Formula **I**. In certain embodiments, R¹ is -O-CH₂CH₃, -O-CH(CH₃)₂, -CH₃, -CH₂CH₃, or -CH₂CH(CH₃)₂.

In certain instances, the co-agent is an inhibitor of a cytochrome p450 2D6 enzyme.

In certain instances, the co-agent is selected from the group consisting of a serotonin norepinephrine reuptake inhibitor; a serotonin noradrenaline dopamine reuptake inhibitor; a norepinephrine dopamine reuptake inhibitor; norfluoxetine, citalopram, dapoxetine, escitalopram, fluvoxamine, paroxetine, sertraline, butriptyline, amoxapine, nortriptyline, clomipramine, desipramine, dosulepin, doxepin, imipramine, dibenzepin, iprindole, lofepramine, opipramol, protriptyline, and trimipramine, or pharmaceutically acceptable salts thereof.

In certain instances, the co-agent is a serotonin norepinephrine reuptake inhibitor selected from the group consisting of venlafaxine, desvenlafaxine, sibutramine, nefazodone, milnacipran, duloxetine, and bicifadine, or pharmaceutically acceptable salts thereof.

In certain instances, the co-agent is a serotonin norepinephrine reuptake inhibitor selected from the group consisting of venlafaxine, desvenlafaxine, nefazodone, and duloxetine, or pharmaceutically acceptable salts thereof.

In certain instances, the co-agent is a serotonin noradrenaline dopamine reuptake inhibitor selected from the group consisting of tesofensine and brasofensine, or pharmaceutically acceptable salts thereof.

In certain instances, the co-agent is a monoamine oxidase inhibitor selected from the group consisting of isocarboxazid, moclobemide, phenelzine, tranylcypromine, selegiline, rasagiline, nialamide, iproniazid, iproclozide, and toloxatone, or pharmaceutically acceptable salts thereof.

In certain instances, the co-agent is a tricyclic antidepressant selected from the group consisting of butriptyline, amoxapine, amitriptyline, nortriptyline, clomipramine, desipramine, dosulepin, doxepin, imipramine, dibenzepin, iprindole, lofepramine, opipramol, protriptyline, and trimipramine, or pharmaceutically acceptable salts thereof.

In certain instances, the co-agent is a tricyclic antidepressant selected from the group consisting of nortriptyline, clomipramine, desipramine, doxepin, and imipramine, or pharmaceutically acceptable salts thereof.

in certain instances, the co-agent is a selective serotonin reuptake inhibitor selected from the group consisting of fluoxetine, norfluoxetine, citalopram, dapoxetine, escitalopram, fluvoxamine, paroxetine, and sertraline, or pharmaceutically acceptable salts thereof.

In certain instances, the co-agent is a selective serotonin reuptake inhibitor selected from the group consisting of citalopram, dapoxetine, escitalopram, fluvoxamine, paroxetine, and sertraline, or pharmaceutically acceptable salts thereof.

In certain instances, the co-agent is paroxetine, or a pharmaceutically acceptable salt thereof.

In another embodiment, provided is a method of treating a neurological disorder selected from the group consisting of amyotrophic lateral sclerosis, multiple sclerosis, Parkinson's disease, Alzheimer's disease, and Huntington's disease in a subject in need thereof, comprising the step of administering to the subject a therapeutically effective amount of a co-agent selected from the grouping consisting of citalopram, fluvoxamine, norfluoxetine, fluoxetine, paroxetine, sertraline, venlafaxine, desvenlafaxine, nefazodone, duloxetine, bupropion, moclobemide, amitriptyline, clomipramine, desipramine, doxepin, imipramine and nortriptyline, or pharmaceutically acceptable salts thereof, and a therapeutically effective amount of a compound selected from the group consisting of: 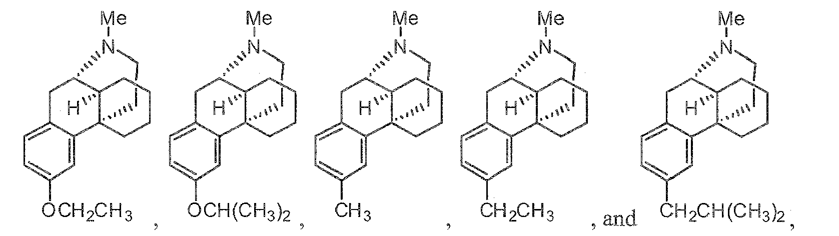 or pharmaceutically acceptable salts thereof.

In another embodiment, provided is a method of treating a neurological disorder selected from the group consisting of amyotrophic lateral sclerosis, multiple sclerosis, Parkinson's disease, Alzheimer's disease, and Huntington's disease in a subject in need thereof, comprising the step of administering to the subject a therapeutically effective amount of a co-agent selected from the grouping consisting of citalopram, fluvoxamine, norfluoxetine, fluoxetine, paroxetine, sertraline, venlafaxine, desvenlafaxine, nefazodone, duloxetine, bupropion, moclobemide, amitriptyline, clomipramine, desipramine, doxepin, imipramine and nortriptyline, or pharmaceutically acceptable salts thereof, and a therapeutically effective amount of a compound with formula: 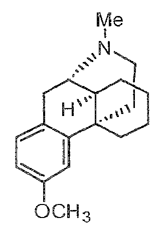 or a pharmaceutically acceptable salt thereof.

In certain instances, the co-agent is citalopram, fluvoxamine, paroxetine, sertraline, venlafaxine, desvenlafaxine, nefazodone, duloxetine, bupropion, moclobemide, clomipramine, desipramine, doxepin, or imipramine, or pharmaceutically acceptable salts thereof.

In certain instances, the co-agent is paroxetine, or a pharmaceutically acceptable salt thereof.

Also provided is a method of treating a brain injury that is the result of stroke, traumatic brain injury, ischemia, hypoglycemia, hypoxia, or neuronal death, comprising the step of administering to the subject a therapeutically effective amount of a co-agent selected from the grouping consisting of a serotonin norepinephrine reuptake inhibitor; a serotonin noradrenaline dopamine reuptake inhibitor; a norepinephrine dopamine reuptake inhibitor; a monoamine oxidase inhibitor; a tricyclic antidepressant; and a selective serotonin reuptake inhibitor; or a pharmaceutically acceptable salts thereof, and a a therapeutically effective amount of a compound selected from the group consisting a compound of Formula **I**: 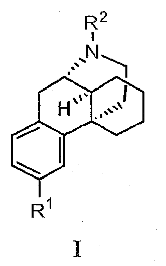 or a pharmaceutically acceptable salt thereof, wherein:
R¹ is selected from -O-(C₂-C₄)akyl and -(C₁-C₄)alkyl; and
R² is -CH₃;
and a compound of Formula **II**: 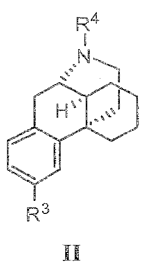 or a pharmaceutically acceptable salt thereof, wherein:
R³ is -OCH₃; and
R⁴ is -CH₃.

In certain embodiments the compound is a compound of Formula **I**. In certain embodiments, R¹ is -O-CH₂CH₃, -O-CH(CH₃)₂, -CH₃, -CH₂CH₃, or -CH₂CH(CH₃)₂.

In certain instances, the co-agent is an inhibitor of a cytochrome p450 2D6 enzyme.

In certain instances, the co-agent is selected from the group consisting of a serotonin norepinephrine reuptake inhibitor; a serotonin noradrenaline dopamine reuptake inhibitor; a norepinephrine dopamine reuptake inhibitor; norfluoxetine, citalopram, dapoxetine, escitalopram, fluvoxamine, paroxetine, sertraline, butriptyline, amoxapine, nortriptyline, clomipramine, desipramine, dosulepin, doxepin, imipramine, dibenzepin, iprindole, lofepramine, opipramol, protriptyline, and trimipramine, or pharmaceutically acceptable salts thereof.

In certain instances, the co-agent is a serotonin norepinephrine reuptake inhibitor selected from the group consisting of venlafaxine, desvenlafaxine, sibutramine, nefazodone, milnacipran, duloxetine, and bicifadine, or pharmaceutically acceptable salts thereof.

In certain instances, the co-agent is a serotonin norepinephrine reuptake inhibitor selected from the group consisting of venlafaxine, desvenlafaxine, nefazodone, and duloxetine, or pharmaceutically acceptable salts thereof.

In certain instances, the co-agent is a serotonin noradrenaline dopamine reuptake inhibitor selected from the group consisting of tesofensine and brasofensine, or pharmaceutically acceptable salts thereof.

In certain instances, the co-agent is a monoamine oxidase inhibitor selected from the group consisting of isocarboxazid, moclobemide, phenelzine, tranylcypromine, selegiline, rasagiline, nialamide, iproniazid, iproclozide, and toloxatone, or pharmaceutically acceptable salts thereof.

In certain instances, the co-agent is a tricyclic antidepressant selected from the group consisting of butriptyline, amoxapine, amitriptyline, nortriptyline, clomipramine, desipramine, dosulepin, doxepin, imipramine, dibenzepin, iprindole, lofepramine, opipramol, protriptyline, and trimipramine, or pharmaceutically acceptable salts thereof.

In certain instances, the co-agent is a tricyclic antidepressant selected from the group consisting of amitriptyline, nortriptyline, clomipramine, desipramine, doxepin, and imipramine, or pharmaceutically acceptable salts thereof.

In certain instances, the co-agent is a selective serotonin reuptake inhibitor selected from the group consisting of fluoxetine, norfluoxetine, citalopram, dapoxetine, escitalopram, fluvoxamine, paroxetine, and sertraline, or pharmaceutically acceptable salts thereof.

In certain instances, the co-agent is a selective serotonin reuptake inhibitor selected from the group consisting of citalopram, dapoxetine, escitalopram, fluvoxamine, paroxetine, and sertraline, or pharmaceutically acceptable salts thereof.

In certain instances, the co-agent is paroxetine, or a pharmaceutically acceptable salt thereof.

In another embodiment, provided is a method of treating treating a brain injury that is the result of stroke, traumatic brain injury, ischemia, hypoglycemia, hypoxia, or neuronal death, in a subject in need thereof, comprising the step of administering to the subject a therapeutically effective amount of a co-agent selected from the grouping consisting of citalopram, fluvoxamine, norfluoxetine, fluoxetine, paroxetine, sertraline, venlafaxine, desvenlafaxine, nefazodone, duloxetine, bupropion, moclobemide, amitriptyline, clomipramine, desipramine, doxepin, imipramine and nortriptyline, or pharmaceutically acceptable salts thereof, and a therapeutically effective amount of a compound selected from the group consisting of: 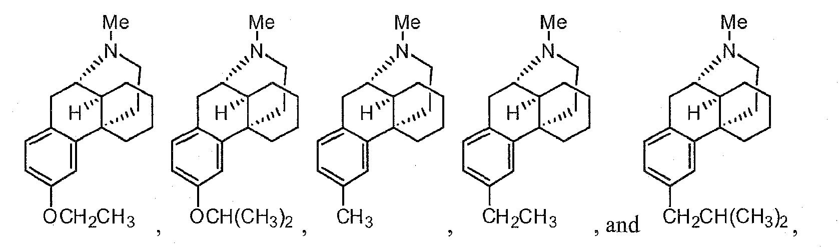 or pharmaceutically acceptable salts thereof.

In another embodiment, provided is a method of treating treating a brain injury that is the result of stroke, traumatic brain injury, ischemia, hypoglycemia, hypoxia, or neuronal death, in a subject in need thereof, comprising the step of administering to the subject a therapeutically effective amount of a co-agent selected from the grouping consisting of citalopram, fluvoxamine, norfluoxetine, fluoxetine, paroxetine, sertraline, venlafaxine, desvenlafaxine, nefazodone, duloxetine, bupropion, moclobemide, amitriptyline, clomipramine, desipramine, doxepin, imipramine and nortriptyline, or pharmaceutically acceptable salts thereof, and a therapeutically effective amount of a compound with formula: 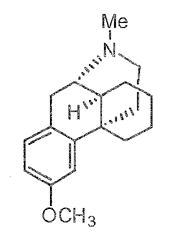 or a pharmaceutically acceptable salt thereof.

In certain instances, the the co-agent is citalopram, fluvoxamine, paroxetine, sertraline, venlafaxine, desvenlafaxine, nefazodone, duloxetine, bupropion, moclobemide, clomipramine, desipramine, doxepin, or imipramine, or pharmaceutically acceptable salts thereof.

In certain instances, the co-agent is paroxetine, or a pharmaceutically acceptable salt thereof.

### DETAILED DESCRIPTION

### Definitions

The terms "ameliorate" and "treat" are used interchangeably and include both therapeutic treatment and/or prophylactic treatment (reducing the likelihood of development). Both terms mean decrease, suppress, attenuate, diminish, arrest, or stabilize the development or progression of a disease (e.g., a disease or disorder delineated herein), lessen the severity of the disease or improve the symptoms associated with the disease.

"Disease" means any condition or disorder that damages or interferes with the normal function of a cell, tissue, or organ.

The term "alkyl" refers to a monovalent saturated hydrocarbon group. C₁-C₄ alkyl is an alkyl having from 1 to 4 carbon atoms. An alkyl may be linear or branched. Examples of alkyl groups include methyl; ethyl; propyl, including n-propyl and isopropyl; butyl, including *n*-butyl, isobutyl, *sec*-butyl, and *t*-butyl.

A salt of a compound provided herein is formed between an acid and a basic group of the compound, such as an amino functional group, or a base and an acidic group of the compound, such as a carboxyl functional group. According to another embodiment, the compound is a pharmaceutically acceptable acid addition salt.

The term "pharmaceutically acceptable," as used herein, refers to a component that is, within the scope of sound medical judgment, suitable for use in contact with the tissues of humans and other mammals without undue toxicity, irritation, allergic response and the like, and are commensurate with a reasonable benefit/risk ratio. A "pharmaceutically acceptable salt" means any suitable salt that, upon administration to a recipient, is capable of providing, either directly or indirectly, a compound provided herein. A "pharmaceutically acceptable counterion" is an ionic portion of a salt that is not toxic when released from the salt upon administration to a recipient.

Acids commonly employed to form pharmaceutically acceptable salts include inorganic acids such as hydrogen bisulfide, hydrochloric acid, hydrobromic acid, hydroiodic acid, sulfuric acid and phosphoric acid, as well as organic acids such as para-toluenesulfonic acid, salicylic acid, tartaric acid, bitartaric acid, ascorbic acid, maleic acid, besylic acid, fumaric acid, gluconic acid, glucuronic acid, formic acid, glutamic acid, methanesulfonic acid, ethanesulfonic acid, benzenesulfonic acid, lactic acid, oxalic acid, para-bromophenylsulfonic acid, carbonic acid, succinic acid, citric acid, benzoic acid and acetic acid, as well as related inorganic and organic acids. Such pharmaceutically acceptable salts thus include sulfate, pyrosulfate, bisulfate, sulfite, bisulfite, phosphate, monohydrogenphosphate, dihydrogenphosphate, metaphosphate, pyrophosphate, chloride, bromide, iodide, acetate, propionate, decanoate, caprylate, acrylate, formate, isobutyrate, caprate, heptanoate, propiolate, oxalate, malonate, succinate, suberate, sebacate, fumarate, maleate, butyne-1,4-dioate, hexyne-1,6-dioate, benzoate, chlorobenzoate, methylbenzoate, dinitrobenzoate, hydroxybenzoate, methoxybenzoate, phthalate, terephthalate, sulfonate, xylene sulfonate, phenylacetate, phenylpropionate, phenylbutyrate, citrate, lactate, β-hydroxybutyrate, glycolate, maleate, tartrate, methanesulfonate, propanesulfonate, naphthalene-1-sulfonate, naphthalene-2- sulfonate, mandelate and other salts. In one embodiment, pharmaceutically acceptable acid addition salts include those formed with mineral acids such as hydrochloric acid and hydrobromic acid, and especially those formed with organic acids such as maleic acid.

The term "stable compounds," as used herein, refers to compounds which possess stability sufficient to allow for their manufacture and which maintain the integrity of the compound for a sufficient period of time to be useful for the purposes detailed herein (e.g., formulation into therapeutic products, intermediates for use in production of therapeutic compounds, isolatable or storable intermediate compounds, treating a disease or condition responsive to therapeutic agents).

"Stereoisomer" refers to both enantiomers and diastereomers. "Tert", " t", and "t" each refer to tertiary. "US" refers to the United States of America. "RT" refers to room temperature. "h" refers to hours. "DMF" refers to dimethylformamide. "TsOH" refers to to p-toluenesulfonic acid.

Throughout this specification, a variable may be referred to generally (e.g.,"each R") or may be referred to specifically (e.g., R¹ or R²). Unless otherwise indicated, when a variable is referred to generally, it is meant to include all specific embodiments of that particular variable.

### Therapeutic Compositions and Methods

Described herein are compositions and methods useful in the treatment of pseudobulbar affect, neuropathic pain, neurodegenerative disease, and brain injuries in a subject in need thereof. In certain instances, treatment comprises the administration of a dextromethorphan analog described herein and an antidepressant. In certain instances, the antidepressant can be a norepinephrine reuptake inhibitor; a serotonin noradrenaline dopamine reuptake inhibitor; a norepinephrine dopamine reuptake inhibitor; a monoamine oxidase inhibitor; a selective serotonin reuptake inhibitor; or a tricyclic antidepressant. The dextromethorphan analogs described herein and the antidepressant can be administered together in a single composition or administered in separate compositions.

In certain instances, the dextromethorphan analog is a compound of Formula **I**: 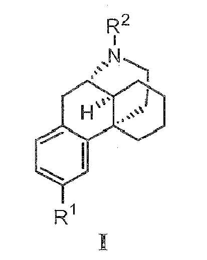 or a pharmaceutically acceptable salt thereof, wherein:
R¹ is -O-(C₂-C₄)alkyl or -(C₁-C₄)akyl; and
R² is -CH₃.

The preferred stereochemistry of the present compounds is based on the stereochemistry of morphinan compounds such as dextromethorphan, which exists as the dextrorotatory enantiomer of levorphanol.

In certain embodiments R¹ is -O-(C₂-C₄)alkyl. In one aspect of this embodiment, R¹ is -O-CH₂CH₃, -O-CH(CH₃)₂, -O-CH₂CH(CH₃)₂.

In another aspect, R¹ is -O-CH₂CH₃ or -O-CH(CH₃)₂. In another aspect, R¹ is -O-CH₂CH₃. In another aspect, R¹ is -O-CH(CH₃)₂.

Each of the above aspects of R¹ may be combined with each of the above aspects of R² to form further embodiments.

Certain embodiments relate to the compound of Formula I, wherein R¹ -(C₁-C₄)alkyl. In one aspect of this embodiment, R¹ is -CH₃, -CH₂CH₃ -CH₂CH₂CH₃, -CH(CH₃)₂,-CH₂CH₂CH₂CH₃, -CH(CH₃)CH₂CH₃, or -CH₂CH(CH₃)₂. Each of these aspects of R¹ may be combined with the below aspects of R² to provide further embodiments.

Examples of specific compounds of Formula I where R¹ is -(C₁-C₄)alkyl include the compounds shown below.

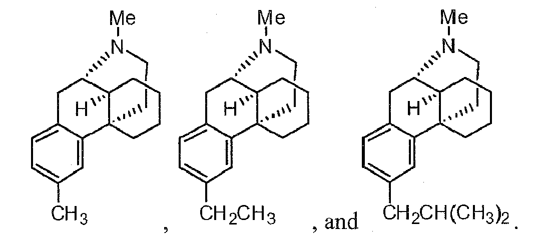

In certain instances the dextromethorphan analog is a compound of Formula **II**: 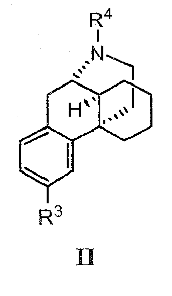 or a pharmaceutically acceptable salt thereof, wherein:
R³ is -OCH₃; and
R⁴ is -CH₃.

The synthesis of compounds of Formula I or Formula II can be readily achieved by synthetic chemists of ordinary skill. Relevant procedures and intermediates are disclosed, for instance, in Schnider, O.; Grussner, A. Helv. Chim. Acta. 1951, 34, p 2211; Grassner, A. & Schnider, O.; GB 713146 (1954); Toyo Pharma K. K., Japan JP 60089474 A (1983); Newman, A. H. et al., J. Med. Chem. 1992, 35, p.4135

In certain instances the co-agent for inclusion in the compositions or use in the methods can be any agent useful in the treatment of pseudobulbar affect, chronic or intractable pain, neurodegenerative disease, or brain injuries. In certain instances the co-agent is an antidepressant. In certain instances the antidepressant is a selective serotonin reuptake inhibitor, a serotonin-norepinephrine reuptake inhibitor, a noradrenergic and specific serotonergic antidepressant, a norepinephrine (noradrenaline) reuptake inhibitor, a norepinephrine-dopamine reuptake inhibitor, tricyclic antidepressant, or a monoamine oxidase inhibitor. In certain instances the co-agent is an inhibitor of a cytochrome p450 2D6 enzyme.

In certain instances the selective serotonin reuptake inhibitor can be citalopram, dapoxetine, escitalopram, fluvoxamine, norfluoxetine, fluoxetine, paroxetine, sertraline, or zimelidine, or pharmaceutically acceptable salts thereof.

In certain instances the serotonin-norepinephrine reuptake inhibitor can be venlafaxine,
desvenlafaxine, sibutramine, nefazodone, milnacipran, duloxetine, or bicifadine, or pharmaceutically acceptable salts thereof.

In certain instances the noradrenergic and specific serotonergic antidepressant can be mirtazapine, or pharmaceutically acceptable salts thereof.

In certain instances the norepinephrine (noradrenaline) reuptake inhibitor can be atomoxetine, reboxetine, viloxazine, maprotiline, bupropion, or radafaxine, or pharmaceutically acceptable salts thereof.

In certain instances the norepinephrine-dopamine reuptake inhibitor can be bupropion, or pharmaceutically acceptable salts thereof.

In certain instances the tricyclic antidepressant can be amitriptyline, butriptyline, amoxapine, clomipramine, desipramine, dosulepin, doxepin, imipramine, dibenzepin, iprindole, lofepramine, nortriptyline, opipramol, protriptyline or trimipramine, or pharmaceutically acceptable salts thereof.

In certain instances the monoamine oxidase inhibitor can be isocarboxazid, moclobemide, phenelzine, tranylcypromine, selegiline, rasagiline, nialamide, iproniazid, iproclozide, or toloxatone, or pharmaceutically acceptable salts thereof.

### Exemplary Synthesis

The co-agents disclosed herein are commercially available or can be prepared using techniques known to those having ordinary skill in the art. Compounds of the disclosed dextromethorphan analog genuses can be prepared by a person skilled in the art using the appropriate reagents and/or intermediates according to the general procedures provided herein and described in the following publications and patents: (Schnider, O., Grussner, A., Helv. Chim. Acta. 1951, 34: 2211; Grussner, A., Schnider, O., GB 713146 (1954); Toyo Pharma K. K., Japan JP 60089474 A (1983); Newman, A. H. et al., J. Med. Chem. 1992, 35: 4135).

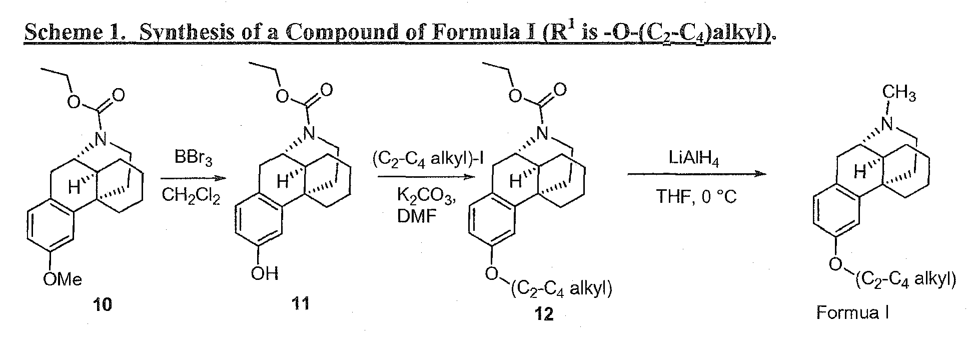

A convenient method for synthesizing compounds of Formula I wherein R¹ is -O-(C₂-C₄)alkyl is depicted in Scheme 1. Treatment of the known 17-ethoxycarbonyl-3-methoxy-morphinan **(10)** with boron tribromide according to the procedure described by Newman, A. H. et al., Journal of Medicinal Chemistry 1992, 35: 4135-4142, affords the 17-ethoxycarbonyl-3-hydroxy-morphinan **(11).** Treatment of the 3-hydroxy-morphinan **11** with the appropriate alkyl iodide in the presence of potassium carbonate according to the procedure described in the aforementioned paper gives the 17-ethoxycarbonyl-3-alkoxy-morphinans (12). Reduction of the carbamate of the morphinan 12 with lithium aluminum in THF affords the 3-alkoxy-17-methyl-morphinan compounds of Formula I.

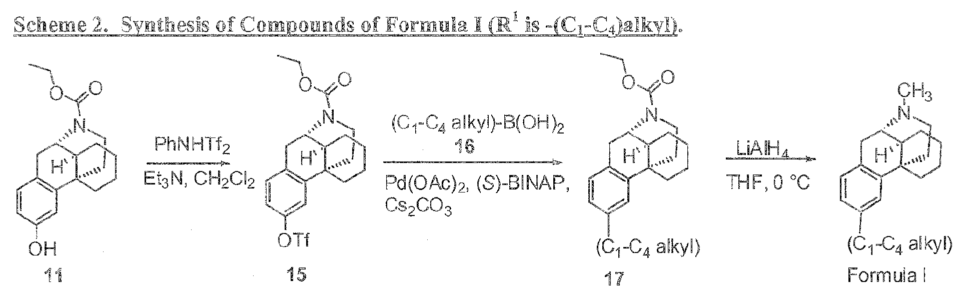

A convenient method for synthesizing compounds of Formula I wherein R¹ is -(C₁-C₄)alkyl is depicted in Scheme 2. Treatment of 17-ethoxycarbonyl-3-hydroxy-morphinan (11) with *N*-Phenyl-trifluoromethanesulfonimide according to the procedure described by Kim, C.-H. in US 2005/0256147 A1 affords the corresponding phenolic triflate (15). Palladium catalyzed cross-coupling of 15 with the appropriate (C₁-C₄)alkyl boronic acid (16) using the procedure from the aforementioned patent gives the 17-ethoxycarbonyl-3-(C₁-C₄)alkyl-morphinans (17). Reduction of the carbamate of morphinan 17 with lithium aluminum hydride in THF according to the procedure described by Newman, A. H. et al., Journal of Medicinal Chemistry 1992, 35: 4135-4142 affords the 3-(C₁-C₄)alkyl-17-methyl-morphinan compounds of Formula I.

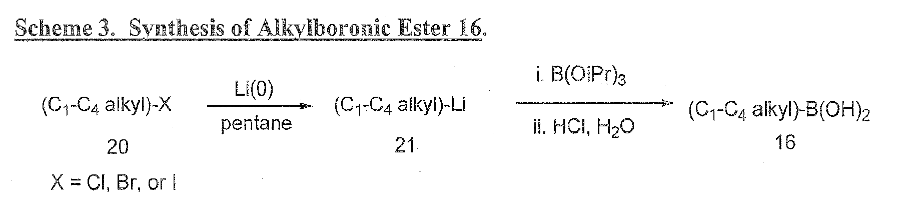

The alkylboronic acid reagent 16 used in Scheme 2 is prepared as described above in Scheme 3. Treatment of the appropriate (C₁-C₄)alkyl halide (20) with elemental lithium in pentane according to the procedure described by Dawildowski, D. et al., in WO 2005/082911 A1 affords the corresponding -(C₁-C₄)alkyl lithium anion, which is immediately treated with triisapropyl borate followed by hydrolysis with aqueous hydrogen chloride according the procedure described by Brown, H. C. et al., Organometallics 1985, 4: 816-821 to afford the appropriate -(C₁-C₄)akyl boronic acids (16).

The compound of Formula II is commercially available.

The specific approaches and compounds shown above are not intended to be limiting. The chemical structures in the schemes herein depict variables that are hereby defined commensurately with chemical group definitions (moieties, atoms, etc.) of the corresponding position in the compound formulae herein, whether identified by the same variable name (i.e., R¹ or R²) or not. The suitability of a chemical group in a compound structure for use in the synthesis of another compound is within the knowledge of one of ordinary skill in the art.

Additional methods of synthesizing compounds of Formula I and their synthetic precursors, including those within routes not explicitly shown in schemes herein, are within the means of chemists of ordinary skill in the art. Synthetic chemistry transformations and protecting group methodologies (protection and deprotection) useful in synthesizing the applicable compounds are known in the art and include, for example, those described in Larock R, Comprehensive Organic Transformations, VCH Publishers (1989); Greene TW et al., Protective Groups in Organic Synthesis, 3rd Ed., John Wiley and Sons (1999); Fieser L et al., Fieser and Fieser's Reagents for Organic Synthesis, John Wiley and Sons (1994); and Paquette L, ed., Encyclopedia of Reagents for Organic Synthesis, John Wiley and Sons (1995) and subsequent editions thereof.

### Compositions

Provided herein are pyrogen-free compositions comprising an effective amount of a compound of Formula **I** or Formula **II** (e.g., including any of the formulae herein), or a pharmaceutically acceptable salt of said compound; a co-agent; and an acceptable carrier. In certain instances the composition is formulated for pharmaceutical use ("a pharmaceutical composition"), wherein the carrier is a pharmaceutically acceptable carrier. The carrier(s) are "acceptable" in the sense of being compatible with the other ingredients of the formulation and, in the case of a pharmaceutically acceptable carrier, not deleterious to the recipient thereof in an amount used in the medicament.

Pharmaceutically acceptable carriers, adjuvants and vehicles that may be used in the pharmaceutical compositions provided herein include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

If required, the solubility and bioavailability of the compounds provided herein in pharmaceutical compositions may be enhanced by methods well-known in the art. One method includes the use of lipid excipients in the formulation. See "Oral Lipid-Based Formulations: Enhancing the Bioavailability of Poorly Water-Soluble Drugs (Drugs and the Pharmaceutical Sciences)," David J. Hauss, ed. Informa Healthcare, 2007; and "Role of Lipid Excipients in Modifying Oral and Parenteral Drug Delivery: Basic Principles and Biological Examples," Kishor M. Wasan, ed. Wiley-Interscience, 2006.

Another known method of enhancing bioavailability is the use of an amorphous form of a compound provided herein optionally formulated with a poloxamer, such as LUTROL™ and PLURONIC™ (BASF Corporation), or block copolymers of ethylene oxide and propylene oxide. See United States patent 7,014,866; and United States patent publications 20060094744 and 20060079502.

The pharmaceutical compositions provided herein include those suitable for oral, rectal, nasal, topical (including buccal and sublingual), vaginal or parenteral (including subcutaneous, intramuscular, intravenous and intradermal) administration. In certain embodiments, the compound of the formulae herein is administered transdermally (e.g., using a transdermal patch or iontophoretic techniques). Other formulations may conveniently be presented in unit dosage form, e.g., tablets, sustained release capsules, and in liposomes, and may be prepared by any methods well known in the art of pharmacy. See, for example, Remington: The Science and Practice of Pharmacy, Lippincott Williams & Wilkins, Baltimore, MD (20th ed. 2000).

Such preparative methods include the step of bringing into association with the molecule to be administered ingredients such as the carrier that constitutes one or more accessory ingredients. In general, the compositions are prepared by uniformly and intimately bringing into association the active ingredients with liquid carriers, liposomes or finely divided solid carriers, or both, and then, if necessary, shaping the product.

In certain embodiments, the compounds are administered orally. The compositions provided herein suitable for oral administration may be presented as discrete units such as capsules, sachets, or tablets each containing a predetermined amount of the active ingredient; a powder or granules; a solution or a suspension in an aqueous liquid or a non-aqueous liquid; an oil-in-water liquid emulsion; a water-in-oil liquid emulsion; packed in liposomes; or as a bolus, etc. Soft gelatin capsules can be useful for containing such suspensions, which may beneficially increase the rate of compound absorption.

In the case of tablets for oral use, carriers that are commonly used include lactose and corn starch. Lubricating agents, such as magnesium stearate, are also typically added. For oral administration in a capsule form, useful diluents include lactose and dried cornstarch. When aqueous suspensions are administered orally, the active ingredient is combined with emulsifying and suspending agents. If desired, certain sweetening and/or flavoring and/or coloring agents may be added.

Compositions suitable for oral administration include lozenges comprising the ingredients in a flavored basis, usually sucrose and acacia or tragacanth; and pastilles comprising the active ingredient in an inert basis such as gelatin and glycerin, or sucrose and acacia.

Compositions suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example, sealed ampules and vials, and may be stored in a freeze dried (lyophilized) condition requiring only the addition of the sterile liquid carrier, for example water for injections, immediately prior to use. Extemporaneous injection solutions and suspensions may be prepared from sterile powders, granules and tablets.

Such injection solutions may be in the form, for example, of a sterile injectable aqueous or oleaginous suspension. This suspension may be formulated according to techniques known in the art using suitable dispersing or wetting agents (such as, for example, Tween 80) and suspending agents. The sterile injectable preparation may also be a sterile injectable solution or suspension in a non-toxic parenterally-acceptable diluent or solvent, for example, as a solution in 1,₃-butanediol. Among the acceptable vehicles and solvents that may be employed are mannitol, water, Ringer's solution and isotonic sodium chloride solution. In addition, sterile, fixed oils are conventionally employed as a solvent or suspending medium. For this purpose, any bland fixed oil may be employed including synthetic mono- or diglycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant.

The pharmaceutical compositions provided herein may be administered in the form of suppositories for rectal administration. These compositions can be prepared by mixing a compound provided herein with a suitable non-irritating excipient which is solid at room temperature but liquid at the rectal temperature and therefore will melt in the rectum to release the active components. Such materials include, but are not limited to, cocoa butter, beeswax and polyethylene glycols.

The pharmaceutical compositions provided herein can be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other solubilizing or dispersing agents known in the art. See, e.g.: Rabinowitz JD and Zaffaroni AC, US Patent 6,803,031, assigned to Alexza Molecular Delivery Corporation.

Topical administration of the pharmaceutical compositions provided herein are useful when the desired treatment involves areas or organs readily accessible by topical application. For topical application topically to the skin, the pharmaceutical composition should be formulated with a suitable ointment containing the active components suspended or dissolved in a carrier. Carriers for topical administration of the compounds provided herein include, but are not limited to, mineral oil, liquid petroleum, white petroleum, propylene glycol, polyoxyethylene polyoxypropylene compound, emulsifying wax, and water. Alternatively, the pharmaceutical composition can be formulated with a suitable lotion or cream containing the active compound suspended or dissolved in a carrier. Suitable carriers include, but are not limited to, mineral oil, sorbitan monostearate, polysorbate 60, cetyl esters wax, cetearyl alcohol, 2-octyldodecanol, benzyl alcohol, and waster. The pharmaceutical compositions provided herein may also be topically applied to the lower intestinal tract by rectal suppository formulation or in a suitable enema formulation.

Application of the subject therapeutics may be local, so as to be administered at the site of interest. Various techniques can be used for providing the subject compositions at the site of interest, such as injection, use of catheters, trocars, projectiles, pluronic gel, stents, sustained drug release polymers or other device which provides for internal access.

Thus, according to yet another embodiment, the compounds provided herein may be incorporated into compositions for coating an implantable medical device, such as prostheses, artificial valves, vascular grafts, stents, or catheters. Suitable coatings and the general preparation of coated implantable devices are known in the art and are exemplified in US Patents 6,099,562; 5,886,026; and 5,304,121. The coatings are typically biocompatible polymeric materials such as a hydrogel polymer, polymethyldisiloxane, polycaprolactone, polyethylene glycol, polylactic acid, ethylene vinyl acetate, and mixtures thereof. The coatings may optionally be further covered by a suitable topcoat of fluorosilicone, polysaccharides, polyethylene glycol, phospholipids or combinations thereof to impart controlled release characteristics in the composition. Coatings for invasive devices are to be included within the definition of pharmaceutically acceptable carrier, adjuvant or vehicle, as those terms are used herein.

According to another embodiment, provided is a method of coating an implantable medical device comprising the step of contacting said device with the coating composition described above. It will be obvious to those skilled in the art that the coating of the device will occur prior to implantation into a mammal.

According to another embodiment, provided is a method of impregnating an implantable drug release device comprising the step of contacting said drug release device with a compound or composition provided herein. Implantable drug release devices include, but are not limited to, biodegradable polymer capsules or bullets, non-degradable, diffusible polymer capsules and biodegradable polymer wafers.

According to another embodiment, provided is an implantable medical device coated with a compound or a composition comprising a compound provided herein, such that said compound is therapeutically active.

According to another embodiment, provided is an implantable drug release device impregnated with or containing a compound or a composition comprising a compound provided herein, such that said compound is released from said device and is therapeutically active.

Where an organ or tissue is accessible because of removal from the subject, such organ or tissue may be bathed in a medium containing a composition provided herein, a composition provided herein may be painted onto the organ, or a composition provided herein may be applied in any other convenient way.

In another embodiment, provided are separate dosage forms of a compound of Formula **I** or Formula **II**; and one or more of any of the above-described co-agents, wherein the compound of Formula **I** or Formula **II**; and the co-agent are associated with one another. The term "associated with one another" as used herein means that the separate dosage forms are packaged together or otherwise attached to one another such that it is readily apparent that the separate dosage forms are intended to be sold and administered together (within less than 24 hours of one another, consecutively or simultaneously).

In the pharmaceutical compositions provided herein, the compound of Formula I or Formula II is present in an effective amount. As used herein, the term "effective amount" refers to an amount which, when administered in a proper dosing regimen, is sufficient to reduce or ameliorate the severity, duration or progression of the disorder being treated, prevent the advancement of the disorder being treated, cause the regression of the disorder being treated, or enhance or improve the prophylactic or therapeutic effect(s) of another therapy.

The interrelationship of dosages for animals and humans (based on milligrams per meter squared of body surface) is described in Freireich et al., (1966) Cancer Chemother. Rep 50:219. Body surface area may be approximately determined from height and weight of the subject. See, e.g., Scientific Tables, Geigy Pharmaceuticals, Ardsley, N.Y., 1970, 537.

In one embodiment, an effective amount of a compound of Formula I or Formula II can range from 0.4 mg to 400 mg, from 4.0 mg to 350 mg, from 10 mg to 90 mg, or from 30 mg to 45 mg, inclusive, which can be given once, twice, or up to three times daily depending on various factors recognized by those skilled in the art.

Effective doses will also vary, as recognized by those skilled in the art, depending on the diseases treated, the severity of the disease, the route of administration, the sex, age and general health condition of the subject, excipient usage, the possibility of co-usage with other therapeutic treatments such as use of other agents and the judgment of the treating physician. For example, guidance for selecting an effective dose can be determined by reference to the prescribing information for dextromethorphan.

An effective amount of the co-agent can be between about 0.01 % to about 100% of the dosage normally utilized in a monotherapy regime using only that agent. The normal monotherapeutic dosages of these co-agents are well known in the art. See, e.g., Wells et al., eds., Pharmacotherapy Handbook, 2nd Edition, Appleton and Lange, Stamford, Conn. (2000); PDR Pharmacopoeia, Tarascon Pocket Pharmacopoeia 2000, Deluxe Edition, Tarascon Publishing, Loma Linda, Calif. (2000), each of which references are incorporated herein by reference in their entirety.

### Methods of Treatment

Provided herein are methods for treating pseudobulbar affect, neuropathic pain, neurodegenerative diseases, and brain injuries in a subject in need thereof, comprising administering a dextromethorphan analog as described herein or a pharmaceutically acceptable salt thereof and a co-agent or a pharmaceutically acceptable salt thereof. In certain instances the co-agent is an antidepressant. In certain instances the co-agent is selected from the group consisting of a serotonin norepinephrine reuptake inhibitor; a serotonin noradrenaline dopamine reuptake inhibitor; a norepinephrine dopamine reuptake inhibitor; a monoamine oxidase inhibitor; a selective serotonin reuptake inhibitor; and a tricyclic antidepressant. In certain instances, the co-agent is also an inhibitor of a p450 2D6 enzyme. Without being bound by theory, the dextromethorphan analogs described herein provide fewer metabolic liabilities, resulting in higher blood levels and/or an increased duration of action. Further, co-administration of an inhibitor of a cytochrome p450 2D6 enzyme with single agent efficacy in the treatment of pseudobulbar affect, neuropathic pain, neurodegenerative diseases, or brain injuries in combination with a dextromethorphan analog (e.g., compounds of Formula **I** and **II**) as described herein can provide increased effectiveness in the treatment of the same.

Provided is a method of treating pseudobulbar affect in a subject in need thereof, comprising the step of administering to the subject a therapeutically effective amount of a co-agent selected from the grouping consisting of a serotonin norepinephrine reuptake inhibitor; a serotonin noradrenaline dopamine reuptake inhibitor; a norepinephrine dopamine reuptake inhibitor; a monoamine oxidase inhibitor; a selective serotonin reuptake inhibitor; and a tricyclic antidepressant; or pharmaceutically acceptable salts thereof, and a therapeutically effective amount of a compound selected from the group consisting a compound of Formula **I**: 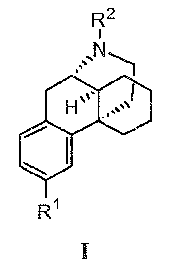 or a pharmaceutically acceptable salt thereof, wherein:
R¹ is selected from -O-(C₂-C₄)alkyl and -(C₁-C₄)alky; and
R² is -CH₃;
and a compound of Formula II: 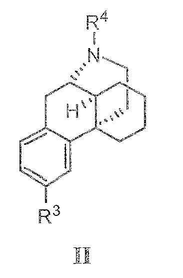 or a pharmaceutically acceptable salt thereof, wherein:
R³ is -OCH₃; and
R⁴ is -CH₃.

In certain embodiments the compound is a compound of Formula **I**. In certain embodiments, R¹ is -O-CH₂CH₃, -O-CH(CH₃)₂, -CH₃, -CH₂CH₃, or -CH₂CH(CH₃)₂.

In other embodiments the compound is a compound of Formula **II**.

In some instances, the co-agents described above are capable of inhibiting the action of a cytochrome p450 2D6 enzyme.

The co-agent can be a serotonin norepinephrine reuptake inhibitor; a serotonin noradrenaline dopamine reuptake inhibitor; a norepinephrine dopamine reuptake inhibitor; norfluoxetine, citalopram, dapoxetine, escitalopram, fluvoxamine, paroxetine, sertraline, butriptyline, amoxapine, amitriptyline, clomipramine, desipramine, dosulepin, doxepin, imipramine, dibenzepin, iprindole, lofepramine, opipramol, protriptyline, and trimipramine, or pharmaceutically acceptable salts thereof.

Serotonin norepinephrine reuptake inhibitors are a class of antidepressants used in the treatment of major depression and other mood disorders. They act upon two neurotransmitters in the brain that are known to play an important part in mood, namely, serotonin and norepinephrine. Serotonin norepinephrine reuptake inhibitors useful as co-agents include venlafaxine, desvenlafaxine, sibutramine, nefazodone, milnacipran, duloxetine, and bicifadine, or pharmaceutically acceptable salts thereof. In some instances the serotonin norepinephrine reuptake inhibitor selected from the group consisting of venlafaxine, desvenlafaxine, nefazodone, and duloxetine, or pharmaceutically acceptable salts thereof.

Serotonin-norepinephrine dopamine reuptake inhibitors are another class of antidepressants that are useful as co-agents in the present disclosure. They act upon neurotransmitters in the brain, namely, serotonin, norepinephrine and dopamine. Serotonin-norepinephrine dopamine reuptake inhibitors are so-called triple reuptake inhibitors, which elevate extracellular plasma concentrations of all three monoamine neurotransmitters, serotonin, norepinephrine and dopamine, in the synaptic cleft. These compounds exhibit low selectivity between the different monoamine transporter proteins. Serotonin noradrenaline dopamine reuptake inhibitors useful as co-agents include tesofensine and brasofensine, or pharmaceutically acceptable salts thereof.

Monoamine oxidase inhibitors are another class of antidepressants useful as co-agents in the present disclosure. Monoamine oxidase inhibitors act by inhibiting the activity of monoamine oxidase preventing the breakdown of monoamine neurotransmitters, which increases their availability. There are two isoforms of monoamine oxidase, MAO-A and MAO-B. MAO-A preferentially deaminates serotonin, melatonin, epinephrine and norepinephrine. MAO-B preferentially deaminates phenylethylamine and trace amines. Dopamine is equally deaminated by both types. Monoamine oxidase inhibitors useful as co-agents in the present disclosure include isocarboxazid, moclobemide, phenelzine, tranylcypromine, selegiline, rasagiline, nialamide, iproniazid, iproclozide, and toloxatone, or pharmaceutically acceptable salts thereof.

Another useful class of co-agents are tricyclic antidepressants. Tricyclic antidepressants are characterized by their molecular structures, which contain three fused ring systems. The tricyclic antidepressants and/or their metabolites act by inhibiting the uptake of one or more monoamine neurotransmitters. Tricyclic antidepressants useful as co-agents in the present disclosure include butriptyline, amoxapine, amitriptyline, nortriptyline, clomipramine, desipramine, dosulepin, doxepin, imipramine, dibenzepin, iprindole, lofepramine, opipramol, protriptyline, and trimipramine, or pharmaceutically acceptable salts thereof. In certain instances, the tricyclic antidepressant is selected from the group consisting of amitriptyline, clomipramine, desipramine, doxepin, and imipramine, or pharmaceutically acceptable salts thereof.

Selective serotonin reuptake inhibitors are also useful as co-agents in the present disclosure. Selective serotonin reuptake inhibitors operate by increasing the extracellular level of the neurotransmitter serotonin by inhibiting its reuptake into the presynaptic cell. They have varying degrees of selectivity for the other monoamine transporters. In general, selective serotonin reuptake inhibitors have little or no binding affinity for the noradrenaline and dopamine transporters. Representative selective serotonin reuptake inhibitors useful as co-agents in the present disclosure include fluoxetine, norfluoxetine, citalopram, dapoxetine, escitalopram, fluvoxamine, paroxetine, and sertraline, or pharmaceutically acceptable salts thereof. In certain instances the selective serotonin reuptake inhibitor is selected from the group consisting of citalopram, norfluoxetine, dapoxetine, escitalopram, fluvoxamine, paroxetine, and sertraline, or pharmaceutically acceptable salts thereof. In one embodiment the co-agent is paroxetine, or a pharmaceutically acceptable salt thereof.

Also provided is a method of treating pseudobulbar affect in a subject in need thereof, comprising the step of administering to the subject a therapeutically effective amount of a co-agent selected from the grouping consisting of citalopram, fluvoxamine, norfluoxetine, fluoxetine, paroxetine, sertraline, venlafaxine, desvenlafaxine, nefazodone, duloxetine, bupropion, moclobemide, amitriptyline, clomipramine, desipramine, doxepin, imipramine and nortriptyline, or pharmaceutically acceptable salts thereof, and a therapeutically effective amount of a dextromethorphan analog. In certain instances the dextromethorphan analog can be any of the following compounds: 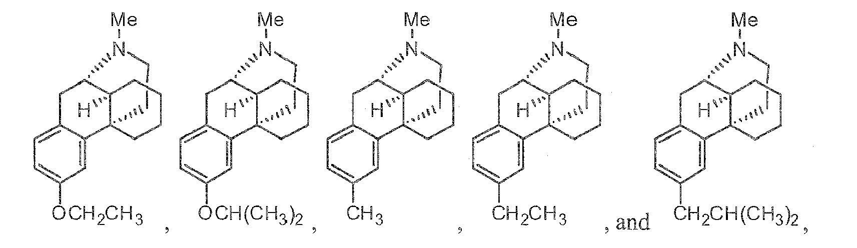 or pharmaceutically acceptable salts thereof.

Also provided is a method of treating pseudobulbar affect in a subject in need thereof, comprising the step of administering to the subject a therapeutically effective amount of a co-agent selected from the grouping consisting of citalopram, fluvoxamine, norfluoxetine, fluoxetine, paroxetine, sertraline, venlafaxine, desvenlafaxine, nefazodone, duloxetine, bupropion, moclobemide, amitriptyline, clomipramine, desipramine, doxepin, imipramine and nortriptyline, or pharmaceutically acceptable salts thereof, and a therapeutically effective amount of a dextromethorphan analog. In certain instances the dextromethorphan analog can be a compound with formula: 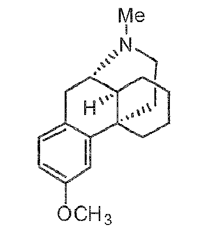 or a pharmaceutically acceptable salt thereof.

In another aspect of the aforementioned embodiment, the co-agent can be citalopram, fluvoxamine, paroxetine, sertraline, venlafaxine, desvenlafaxine, nefazodone, duloxetine, bupropion, moclobemide, clomipramine, desipramine, doxepin, or imipramine, or a pharmaceutically acceptable salts thereof. In yet another aspect of the aforementioned embodiment, the co-agent is paroxetine, or a pharmaceutically acceptable salt thereof.

Also provided is a method of treating chronic or intractable pain. Chronic or intractable pain includes pain related to stroke, trauma, cancer, cancer treatment, fibromyalgia, and pain due to neuropathies such as herpes zoster infection (*i.e.*, postherpetic neurgalia), and diabetes (diabetic neuropathy). Neuropathic pain also includes phantom limb pain, trigeminal neuralgia, and sciatica. The method comprises the step of administering to a subject a therapeutically effective amount of a co-agent and a dextromethorphan analog. The co-agent can be an antidepressant. In certain instances the co-agent can be a serotonin norepinephrine reuptake inhibitor; a serotonin noradrenaline dopamine reuptake inhibitor; a norepinephrine dopamine reuptake inhibitor; a monoamine oxidase inhibitor; a tricyclic antidepressant; or a selective serotonin reuptake inhibitor; or pharmaceutically acceptable salts thereof. The dextromethorphan analog is selected from the group consisting of a compound of Formula I: 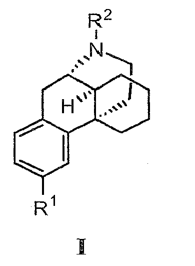 or a pharmaceutically acceptable salt thereof, wherein:
R¹ is selected from -O-(C₂-C₄)alkyl and -(C₁-C₄)alky; and
R² is -CH₃;
and a compound of Formula **II**: 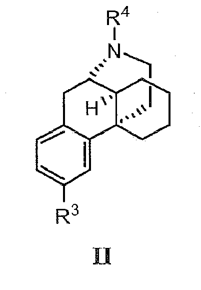 or a pharmaceutically acceptable salt thereof, wherein:
R³ is -OCH₃; and
R⁴ is -CH₃.

In certain embodiments the compound is a compound of Formula **I**. In certain embodiments, R¹ is -O-CH₂CH₃, -O-CH(CH₃)₂, -CH₃, -CH₂CH₃, or -CH₂CH(CH₃)₂.

In other embodiments the compound is a compound of Formula **II**.

In some instances, the co-agents described above are capable of inhibiting the action of a cytochrome p450 2D6 enzyme.

In certain instances the co-agent can be a serotonin norepinephrine reuptake inhibitor; a serotonin noradrenaline dopamine reuptake inhibitor; a norepinephrine dopamine reuptake inhibitor; norfluoxetine, citalopram, dapoxetine, escitalopram, fluvoxamine, paroxetine, sertraline, butriptyline, amoxapine, amitriptyline, clomipramine, desipramine, dosulepin, doxepin, imipramine, dibenzepin, iprindole, lofepramine, opipramol, protriptyline, and trimipramine, or pharmaceutically acceptable salts thereof.

In certain instances the co-agent can be a serotonin norepinephrine reuptake inhibitor. The serotonin norepinephrine reuptake inhibitor can be venlafaxine, desvenlafaxine, sibutramine, nefazodone, milnacipran, duloxetine, or bicifadine, or pharmaceutically acceptable salts thereof. In certain instances the co-agent is a serotonin norepinephrine reuptake inhibitor selected from the group consisting of venlafaxine, desvenlafaxine, nefazodone, and duloxetine, or pharmaceutically acceptable salts thereof.

In certain instances the co-agent can be a serotonin noradrenaline dopamine reuptake inhibitor. The serotonin noradrenaline dopamine reuptake inhibitor can be tesofensine and brasofensine, or pharmaceutically acceptable salts thereof.

Monoamine oxidase inhibitors are also useful as co-agents in the aforementioed method. The monoamine oxidase inhibitor can be isocarboxazid, moclobemide, phenelzine, tranylcypromine, selegiline, rasagiline, nialamide, iproniazid, iproclozide, or toloxatone, or pharmaceutically acceptable salts thereof.

In certain embodiments, the co-agent is a tricyclic antidepressant. The tricyclic antidepressant can be butriptyline, amoxapine, amitriptyline, nortriptyline, clomipramine, desipramine, dosulepin, doxepin, imipramine, dibenzepin, iprindole, lofepramine, opipramol, protriptyline, or trimipramine, or pharmaceutically acceptable salts thereof. In another instance the the tricyclic antidepressant is selected from the group consisting of amitriptyline, clomipramine, desipramine, doxepin, and imipramine, or pharmaceutically acceptable salts thereof.

The co-agent can be a selective serotonin reuptake inhibitor. The selective serotonin reuptake inhibitor can be fluoxetine, norfluoxetine, citalopram, dapoxetine, escitalopram, fluvoxamine, paroxetine, or sertraline, or pharmaceutically acceptable salts thereof. In certain instances the co-agent is citalopram, norfluoxetine, dapoxetine, escitalopram, fluvoxamine, paroxetine, or sertraline, or pharmaceutically acceptable salts thereof. In another embodiment the co-agent is paroxetine, or a pharmaceutically acceptable salt thereof.

Also provided is a method of treating chronic or intractable pain in a subject in need thereof, comprising the step of administering to the subject a therapeutically effective amount of a co-agent selected from the grouping consisting of citalopram, fluvoxamine, norfluoxetine, fluoxetine, paroxetine, sertraline, venlafaxine, desvenlafaxine, nefazodone, duloxetine, bupropion, moclobemide, amitriptyline, clomipramine, desipramine, doxepin, imipramine and nortriptyline, or pharmaceutically acceptable salts thereof, and a therapeutically effective amount of a compound selected from the group consisting of: 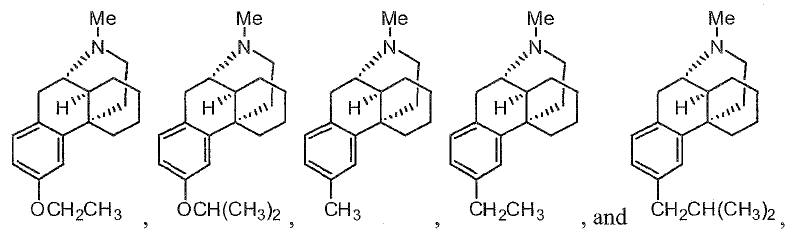 or pharmaceutically acceptable salts thereof.

Also provided is a method of treating chronic or intractable pain in a subject in need thereof, comprising the step of administering to the subject a therapeutically effective amount of a co-agent selected from the grouping consisting of citalopram, fluvoxamine, norfluoxetine, fluoxetine, paroxetine, sertraline, venlafaxine, desvenlafaxine, nefazodone, duloxetine, bupropion, moclobemide, amitriptyline, clomipramine, desipramine, doxepin, imipramine and nortriptyline, or pharmaceutically acceptable salts thereof, and a therapeutically effective amount of a compound with formula: 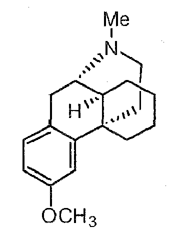 or pharmaceutically a acceptable salt thereof.

Another embodiment relates to the aforementioned method where in the co-agent can be citalopram, fluvoxamine, paroxetine, sertraline, venlafaxine, desvenlafaxine, nefazodone, duloxetine, bupropion, moclobemide, clomipramine, desipramine, doxepin, or imipramine, or pharmaceutically acceptable salts thereof. The co-agent can also be paroxetine, or a pharmaceutically acceptable salt thereof.

Another embodiment relates to any of the aforementioned methods for treating chronic or intractable pain, where the chronic or intractable pain is a neuropathic pain.

Another embodiment relates to any of the aforementioned methods for treating chronic or intractable pain, where the chronic or intractable pain is diabetic neuropathic pain.

In another embodiment, provided is a method of treating a neurological disorder. The neurological disorder can be amyotrophic lateral sclerosis, multiple sclerosis, Parkinson's disease, Alzheimer's disease, or Huntington's disease. The method comprises the step of administering to a subject, in need thereof, a therapeutically effective amount of a co-agent and a a dextromethorphan analog. In certain instances the co-agent is an antidepressant. In certain instances the antidepressant can be a serotonin norepinephrine reuptake inhibitor; a serotonin noradrenaline dopamine reuptake inhibitor; a norepinephrine doparamine reuptake inhibitor; a monoamine oxidase inhibitor; a tricyclic antidepressant; or a selective serotonin reuptake inhibitor; or pharmaceutically acceptable salts thereof. The dextromethorphan analog is selected from the group consisting of a compound of Formula I: 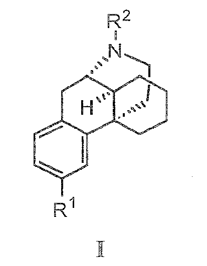 or a pharmaceutically acceptable salt thereof, wherein:
R¹ is selected from -O-(C₂-C₄)alkyl and -(C₁-C₄)alky; and
R² is -CH₃;
and a compound of Formula II: 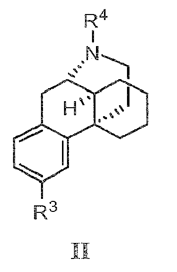 or a pharmaceutically acceptable salt thereof, wherein:
R³ is -OCH₃; and
R⁴ is -CH₃.

In certain embodiments the compound is a compound of Formula **I**. In certain embodiments, R¹ is -O-CH₂CH₃, -O-CH(CH₃)₂, -CH₃, -CH₂CH₃, or -CH₂CH(CH₃)₂.

In other embodiments the compound is a compound of Formula **II**.

In some instances, the co-agents described above are capable of inhibiting the action of a cytochrome p450 2D6 enzyme.

In certain instances, the co-agent can be a serotonin norepinephrine reuptake inhibitor; a serotonin noradrenaline dopamine reuptake inhibitor; a norepinephrine dopamine reuptake inhibitor; norfluoxetine, citalopram, dapoxetine, escitalopram, fluvoxamine, paroxetine, sertraline, butriptyline, amoxapine, nortriptyline, clomipramine, desipramine, dosulepin, doxepin, imipramine, dibenzepin, iprindole, lofepramine, opipramol, protriptyline, and trimipramine, or pharmaceutically acceptable salts thereof. In certain instances the serotonin norepinephrine reuptake inhibitor is venlafaxine, desvenlafaxine, sibutramine, nefazodone, milnacipran, duloxetine, or bicifadine, or pharmaceutically acceptable salts thereof. In certain instances the serotonin norepinephrine reuptake inhibitor selected from the group consisting of venlafaxine, desvenlafaxine, nefazodone, and duloxetine, or pharmaceutically acceptable salts thereof.

Serotonin noradrenaline dopamine reuptake inhibitors are also useful as co-agents. The serotonin noradrenaline dopamine reuptake inhibitors can be tesofensine or brasofensine, or pharmaceutically acceptable salts thereof.

In certain instances, the co-agent is a monoamine oxidase inhibitor. The monoamine oxidase inhibitor can be isocarboxazid, moclobemide, phenelzine, tranylcypromine, selegiline, rasagiline, nialamide, iproniazid, iproclozide, and toloxatone, or pharmaceutically acceptable salts thereof.

In certain instances the co-agent is a tricyclic antidepressant selected from the group consisting of butriptyline, amoxapine, amitriptyline, nortriptyline, clomipramine, desipramine, dosulepin, doxepin, imipramine, dibenzepin, iprindole, lofepramine, opipramol, protriptyline, and trimipramine, or pharmaceutically acceptable salts thereof. In some embodiments the tricyclic antidepressant is nortriptyline, clomipramine, desipramine, doxepin, or imipramine, or pharmaceutically acceptable salts thereof.

Selective serotonin reuptake inhibitors are also useful as co-agents. The selective serotonin reuptake inhibitor can be fluoxetine, norfluoxetine, citalopram, dapoxetine, escitalopram, fluvoxamine, paroxetine, or sertraline, or pharmaceutically acceptable salts thereof. In certain instances the selective serotonin reuptake inhibitor is citalopram, dapoxetine, escitalopram, fluvoxamine, paroxetine, and sertraline, or pharmaceutically acceptable salts thereof. In one embodiment the co-agent is paroxetine, or a pharmaceutically acceptable salt thereof.

Also provided is a method of treating a neurological disorder. The neurological disorder can be amyotrophic lateral sclerosis, multiple sclerosis, Parkinson's disease, Alzheimer's disease, or Huntington's disease. The method for treating the neurological disorder comprises the step of administering to a subject, in need thereof, a therapeutically effective amount of a dextromethorphan analog and a co-agent. In certain instances, the co-agent is citalopram, fluvoxamine, norfluoxetine, fluoxetine, paroxetine, sertraline, venlafaxine, desvenlafaxine, nefazodone, duloxetine, bupropion, moclobemide, amitriptyline, clomipramine, desipramine, doxepin, imipramine or nortriptyline, or pharmaceutically acceptable salts thereof. The dextromethorphan analog can be an analog selected from the group consisting of: 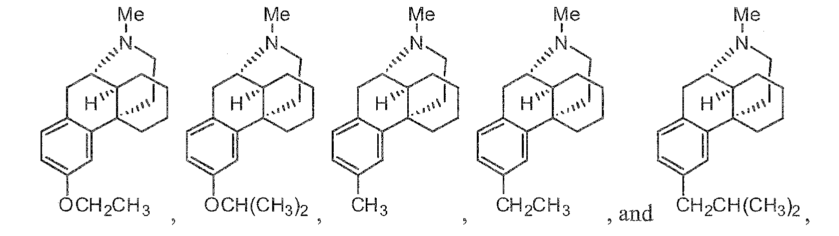 or pharmaceutically acceptable salts thereof.

Also provided is a method of treating a neurological disorder. The neurological disorder can be amyotrophic lateral sclerosis, multiple sclerosis, Parkinson's disease, Alzheimer's disease, or Huntington's disease. The method for treating the neurological disorder comprises the step of administering to a subject, in need thereof, a therapeutically effective amount of a dextromethorphan analog and a co-agent. In certain instances, the co-agent is citalopram, fluvoxamine, norfluoxetine, fluoxetine, paroxetine, sertraline, venlafaxine, desvenlafaxine, nefazodone, duloxetine, bupropion, moclobemide, amitriptyline, clomipramine, desipramine, doxepin, imipramine or nortriptyline, or pharmaceutically acceptable salts thereof. The dextromethorphan analog can be a compound with formula: 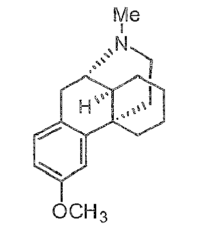 or a pharmaceutically acceptable salt thereof.

In certain instances the co-agent can be citalopram, fluvoxamine, paroxetine, sertraline, venlafaxine, desvenlafaxine, nefazodone, duloxetine, bupropion, moclobemide, clomipramine, desipramine, doxepin, or imipramine, or pharmaceutically acceptable salts thereof. In certain instances the co-agent is paroxetine, or a pharmaceutically acceptable salt thereof.

Also provided is a method of treating a brain injury. The brain injury can be the result of a stroke, a traumatic brain injury, ischemia, hypoglycemia, hypoxia, or neuronal death. The method of treating the brain injury comprises the step of administering to a subject, in need thereof, a therapeutically effective amount of a co-agent selected from the grouping consisting of a serotonin norepinephrine reuptake inhibitor; a serotonin noradrenaline dopamine reuptake inhibitor; a norepinephrine dopamine reuptake inhibitor; a monoamine oxidase inhibitor; a tricyclic antidepressant; and a selective serotonin reuptake inhibitor; or a pharmaceutically acceptable salts thereof, and a therapeutically effective amount of a compound selected from the group consisting of compounds of Formula I: 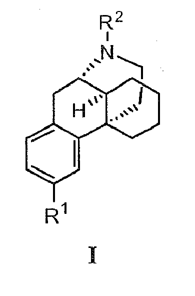 or a pharmaceutically acceptable salt thereof, wherein:
R¹ is selected from -O-(C₂-C₄)alkyl and -(C₁₋C₄)alkyl; and
R² is -CH₃;
and a compound of Formula II: 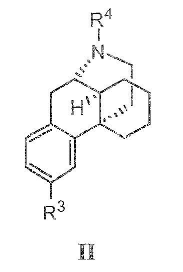 or a pharmaceutically acceptable salt thereof, wherein:
R³ is -OCH₃; and
R⁴ is -CH₃.

In certain embodiments the compound is a compound of Formula I. In certain embodiments, R¹ is -O-CH₂CH₃, -O-CH(CH₃)₂, -CH₃, -CH₂CH₃, or -CH₂CH(CH₃)_{2.}

In other embodiments the compound is a compound of Formula II.

In some instances, the co-agents described above are capable of inhibiting the action of a cytochrome p450 2D6 enzyme.

The co-agent can be a serotonin norepinephrine reuptake inhibitor; a serotonin noradrenaline dopamine reuptake inhibitor; a norepinephrine dopamine reuptake inhibitor; norfluoxetine, citalopram, dapoxetine, escitalopram, fluvoxamine, paroxetine, sertraline, butriptyline, amoxapine, nortriptyline, clomipramine, desipramine, dosulepin, doxepin, imipramine, dibenzepin, iprindole, lofepramine, opipramol, protriptyline, or trimipramine, or pharmaceutically acceptable salts thereof. The serotonin norepinephrine reuptake inhibitor can also be venlafaxine, desvenlafaxine, sibutramine, nefazodone, milnacipran, duloxetine, or bicifadine, or pharmaceutically acceptable salts thereof. The co-agent can also be a serotonin norepinephrine reuptake inhibitor selected from the group consisting of venlafaxine, desvenlafaxine, nefazodone, and duloxetine, or pharmaceutically acceptable salts thereof.

Serotonin noradrenaline dopamine reuptake inhibitor are also useful as co-agents. The serotonin noradrenaline dopamine reuptake inhibitor can be tesofensine or brasofensine, or pharmaceutically acceptable salts thereof.

The co-agent can also be a monoamine oxidase inhibitor selected from the group consisting of isocarboxazid, moclobemide, phenelzine, tranylcypromine, selegiline, rasagiline, nialamide, iproniazid, iproclozide, and toloxatone, or pharmaceutically acceptable salts thereof.

The co-agent can also be a tricyclic antidepressant selected from the group consisting of butriptyline, amoxapine, amitriptyline, nortriptyline, clomipramine, desipramine, dosulepin, doxepin, imipramine, dibenzepin, iprindole, lofepramine, opipramol, protriptyline, and trimipramine, or pharmaceutically acceptable salts thereof. In certain instances, the tricyclic antidepressant is amitriptyline, nortriptyline, clomipramine, desipramine, doxepin, and imipramine, or pharmaceutically acceptable salts thereof.

The co-agent can also be a selective serotonin reuptake inhibitor selected from the group consisting of fluoxetine, norfluoxetine, citalopram, dapoxetine, escitalopram, fluvoxamine, paroxetine, and sertraline, or pharmaceutically acceptable salts thereof. In some instances, the selective serotonin reuptake inhibitor selected from the group consisting of citalopram, dapoxetine, escitalopram, fluvoxamine, paroxetine, and sertraline, or pharmaceutically acceptable salts thereof. The co-agent can also be paroxetine, or a pharmaceutically acceptable salt thereof.

Also provided is a method of treating a brain injury. The brain injury can be the result of a stroke, a traumatic brain injury, ischemia, hypoglycemia, hypoxia, or neuronal death. The method of treating the brain injury comprises the step of administering to a subject, in need thereof, a therapeutically effective amount of a co-agent selected from the grouping consisting of citalopram, fluvoxamine, norfluoxetine, fluoxetine, paroxetine, sertraline, venlafaxine, desvenlafaxine, nefazodone, duloxetine, bupropion, moclobemide, amitriptyline, clomipramine, desipramine, doxepin, imipramine and nortriptyline, or pharmaceutically acceptable salts thereof, and a therapeutically effective amount of a compound selected from the group consisting of: 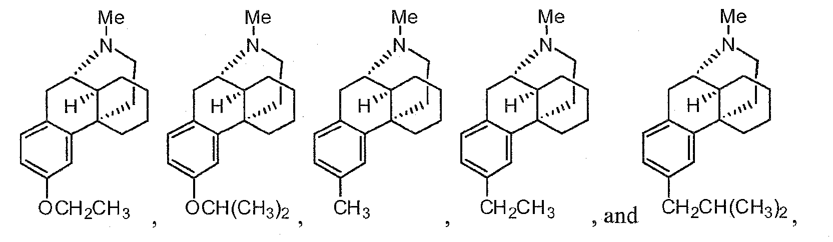 or pharmaceutically acceptable salts thereof.

Also provided is a method of treating a brain injury. The brain injury can be the result of a stroke, a traumatic brain injury, ischemia, hypoglycemia, hypoxia, or neuronal death.

The method of treating the brain injury comprises the step of administering to a subject, in need thereof, a therapeutically effective amount of a co-agent selected from the grouping consisting of citalopram, fluvoxamine, norfluoxetine, fluoxetine, paroxetine, sertraline, venlafaxine, desvenlafaxine, nefazodone, duloxetine, bupropion, moclobemide, amitriptyline, clomipramine, desipramine, doxepin, imipramine and nortriptyline, or pharmaceutically acceptable salts thereof, and a therapeutically effective amount of a compound with formula: 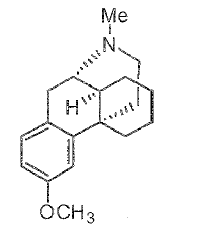 or a pharmaceutically acceptable salt thereof.

In certain instances the co-agent can be citalopram, fluvoxamine, paroxetine, sertraline, venlafaxine, desvenlafaxine, nefazodone, duloxetine, bupropion, moclobemide, clomipramine, desipramine, doxepin, or imipramine, or pharmaceutically acceptable salts thereof. The co-agent can also be paroxetine, or a pharmaceutically acceptable salt thereof.

The co-agent may be administered together with a compound of Formula I or Formula II as part of a single dosage form or as separate, multiple dosage forms. Alternatively, the co-agent may be administered prior to, consecutively with, or following the administration of a compound of Formula I or Formula II. In such combination therapy treatment, both the compound of Formula I or Formula II; and the co-agent are administered by conventional methods.

Effective amounts of the co-agent are well known to those skilled in the art and guidance for dosing may be found in patents and published patent applications referenced herein, as well as in Wells et al., eds., Pharmacotherapy Handbook, 2nd Edition, Appleton and Lange, Stamford, Conn. (2000); PDR Pharmacopoeia, Tarascon Pocket Pharmacopoeia 2000, Deluxe Edition, Tarascon Publishing, Loma Linda, Calif. (2000), and other medical texts. However, it is well within the skilled artisan's purview to determine the co- agent's optimal effective-amount range.

In certain embodiments, the effective amount of the co-agent is less than its effective amount would be where the compound of Formula I or Formula II is not administered. In this way, undesired side effects associated with high doses of either agent may be minimized. Other potential advantages (including without limitation improved dosing regimens and/or reduced drug cost) will be apparent to those of skill in the art.

Certain embodiments relate to any of the aforementioned methods, where an effective amount of a compound of Formula I or Formula II can range from about 0.4 mg to about 400 mg, from about 4.0 mg to about 350 mg, from about 10 mg to about 250 mg, from about 10 mg to about 150 mg, from about 10 mg to about 90 mg, from about 1 mg to about 60 mg, from about 10 mg to about 40 mg, from about 20 mg to about 30 mg, or from about 30 mg to about 45 mg. The dose be given once, twice, or up to three times daily depending on various factors recogonized by those skilled in the art.

Certain embodiments relate to any of the aforementioned methods, where the co-agent is paroxetine. In certain instances, an effective amount of paroxetine, when dosed with a compound of Formula I or Formula **II,** can range from about 1 mg to about 40 mg, from about 1 mg to about 30 mg, from about 5 mg to about 40 mg, from about 5 mg to about 25 mg, from about 10 mg to about 40 mg, from about 10 mg to about 20 mg, from about 15 mg to about 40 mg, from about 20 mg to about 40 mg, from about 20 mg to about 35 mg, or from about 25 mg to about 35 mg.

In yet another aspect, provided is the use of a compound of Formula **I or** Formula II together with one or more of the above-described co-agents in the manufacture of a medicament, either as a single composition or as separate dosage forms, for treatment or prevention in a subject of a disease, disorder or symptom set forth above.

### Kits

Also provided are kits for use to treat pseudobulbar disorder, chronic or intractable pain, neurodegenerative diseases, or brain injuries. These kits comprise (a) a pharmaceutical composition comprising a compound of Formula **I** or Formula **II** and a co-agent, as described above, or pharmaceutically acceptable salts thereof, wherein said pharmaceutical composition is in a container; and (b) instructions describing a method of using the pharmaceutical composition to treat pseudobulbar disorder, chronic or intractable pain, a neurodegenerative disease, or a brain injury.

In certain embodiments, the kits comprise (a) a first pharmaceutical composition comprising a compound of Formula **I** or Formula **II** or pharmaceutically acceptable salts thereof; (b) a second pharmaceutical composition comprising a co-agent as described above or a pharmaceutically acceptable salt thereof; wherein the first pharmaceutical composition and the second pharmaceutical composition are contained in separate containers; and (c) instructions describing a method of using the first pharmaceutical composition and the second pharmaceutical composition to treat pseudobulbar disorder, chronic or intractable pain, a neurodegenerative disease, or a brain injury.

The container(s) may be any vessel or other sealed or sealable apparatus that can hold said pharmaceutical composition(s). Examples include bottles, ampules, divided or multi-chambered holders bottles, wherein each division or chamber comprises a single dose of said composition, a divided foil packet wherein each division comprises a single dose of said composition, or a dispenser that dispenses single doses of said composition. The container can be in any conventional shape or form as known in the art which is made of a pharmaceutically acceptable material, for example a paper or cardboard box, a glass or plastic bottle or jar, a re-sealable bag (for example, to hold a "refill" of tablets for placement into a different container), or a blister pack with individual doses for pressing out of the pack according to a therapeutic schedule. The container employed can depend on the exact dosage form involved, for example a conventional cardboard box would not generally be used to hold a liquid suspension. It is feasible that more than one container can be used together in a single package to market a single dosage form. For example, tablets may be contained in a bottle, which is in turn contained within a box. In on embodiment, the container is a blister pack.

The kits may also comprise a device to administer or to measure out a unit dose of the pharmaceutical composition. Such device may include an inhaler if said composition is an inhalable composition; a syringe and needle if said composition is an injectable composition; a syringe, spoon, pump, or a vessel with or without volume markings if said composition is an oral liquid composition; or any other measuring or delivery device appropriate to the dosage formulation of the composition present in the kit.

### Examples

### Example 1. Synthesis of (+)-3-Ethoxy-17-methyl-(9α,13α,14α)-morphinan hydrochloride (100). Compound 100 can be prepared as outlined in Scheme 4 below. Details of a synthesis follows.

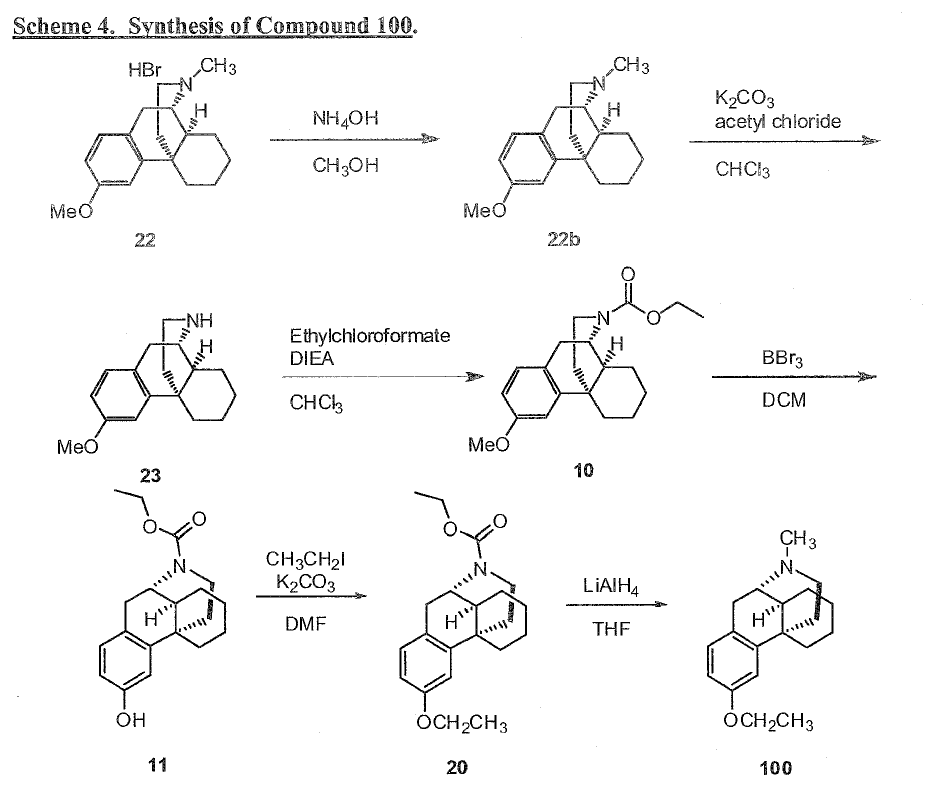

### Synthesis of (+)-3-methoxy-17-methyl-(9α,13α,14α)-morphinan (free base, 22b).

To a reaction vessel is added (+)-3-methoxy-17-methyl-(9α,13α,14α)-morphinan, HBr salt (22; 3.00g, 8.5 mmol), NH₃ in CH₃OH (2.0 M, 8.5 mL, 17.0 mmol), and a stir bar. The reaction mixture is stirred at RT for 1 h. The resulting material is concentrated on a rotary evaporator, then diluted with CHCl₃ (50 mL) and H₂O (50 mL). The layers are separated and the water layer is extracted with CHCl₃ (50 mL). The combined organic layers are dried over magnesium sulfate, filtered and concentrated on a rotary evaporator to yield **22b.**

**Synthesis of (+)-3-methoxy-(9α,13α,14α)-morphinan (23)**. The solid (+)-3-methoxy-17-methyl-(9α,13α,14α)-morphinan (22b; 6.79 g, 25.1 mmol) is placed in a reaction vessel with CHCl₃ and a stir bar. K₂CO₃ (13.85 g, 100.2 mmol) is added and the mixture is stirred at RT under an atmosphere of N₂ for 10 min before the addition of acetyl chloride (7.866 g, 100.2 mmol). The resulting reaction mixture, still under an atmosphere of N₂, is stirred under reflux conditions for 7 h, then filtered throngh a pad of celite. The organic filtrate is concentrated on a rotary evaporator and the resulting crude material is dissolved in CH₃OH then stirred under reflux conditions for 1 h. The solution is concentrated on a rotary evaporator then dried under vacuum to yield 23.

**Synthesis of (+)-17-ethylcarbamate-3-methoxy-(9α,13α,14α)-morphinan (10).** To a reaction vessel fit with a stirbar is added 23 (6.025g, 2.48 mmol) dissolved in CHCl₃ (100 mL). Diisopropylethylamine (DIEA; 16.32 g, 126.3 mmol) is added and the mixture is stirred for 10 min at room temperature under nitrogen before the addition of ethylchloroformate (13.094 g, 76.8 mmol). The reaction mixture is stirred under reflux conditions under nitrogen for 3 h, at which point TLC (20% ethylacetate/hexane) showed complete consumption of the starting material. The organic layer is remove and washed first with 1M HCl, and then with saturated NaHCO₃. The aqueous layers from each wash are combined and back extracted with 50 ml of CHCl₃ . The organic layer from the back extraction is combined with the organic layer from the washes and the combined organic layers are dried over Na₂SO₄. The organic solution is then filtered, concentrated on a rotary evaporator then is purified via automated flash column chromatography (0-30% ethylacetate/hexane) to yield 10.

**(+)-17-ethylcarbamate-3-hydroxy-(9α,13α,14α)-morphinan(11)**. In a reaction vessel fit with a stirbar the carbamate 10 (2.43 g, 7.4 mmol) is dissolved in DCM (20 mL) and the resulting solution is cooled to 0°C. BBr₃ (9.24 g, 36.9 mmol) is added and the reaction mixture is stirred under an atmosphere of N₂ at 0 °C for 20 min (at which time tlc in 20% ethylacetate/hexane showed the reaction to be complete). A solution of 27% NH₄OH in ice is placed in a beaker with a stir bar and the reaction mixture is slowly added with stirring. The resulting mixture is stirred for 20 min then is extracted with 4:1 CHCl₃/CH₃OH (200 mL). The organic layer is dried over Na₂SO₄, filtered, then concentrated on a rotary evaporator. The crude material is purified via automated flash column chromatography (CH₃OH with 1% NH₄OH / CHCl₃, 0-10%). The pure fractions are concentrated on a rotary evaporator to yield 11.

**Synthesis of (+)-3-ethoxy-17-ethoxycarbonyl-(9α,13α,14α)-morphinan (20)**. To a solution of alcohol 11 (1.50 g, 4.8 mmol) in DMF (25 mL), is added K₂CO₃ (2.00 g, 14.5 mmol, 3.05 eq) and iodoethane (1.15 g, 7.1 mmol, 1.50 eq) with stirring. The reaction mixture is stirred overnight at room temperature under an atmosphere of N₂, then quenched by the addition of H₂O, and extracted with Et₂O (3 x 30 mL). The combined organics are dried over Na₂SO₄, filtered and concentrated *in vacuo* to a yellow oil. Purification via automated flash column chromatography (0-40% EtOAc/hexanes) affordeds intermediate **20.**

**Synthesis of (+)-3-ethoxy-17-methyl-(9α,13α,14α)-morphinan hydrochloride** (100). To a slurry of LiAlH₄ (0.184 g, 4.4 mmol, 2.0 eq) in THF (10 mL) stirring at -78°C is added a solution of the carbamate 20 (0.763 g, 2.2 mmol) in THF (5 mL). After 1 h of stirring at rt, an additional 2.0 eq of LiAlH₄ (0.184 g, 4.4 mmol, 2.0 eq) is added. The reaction mixture is stirred overnight at rt, then quenched by the addition of magnesium sulfate heptahydrate until cessation of gas evolution. The mixture is filtered, concentrated in *vacuo* and the resultant crude material is purified via automated flash column chromatography (CHCl₃/CH₃OH/NH₃OH - 90/10/1) to yield the free amine 100. This material is dissolved in 1.25 M HCl in CH₃OH then is concentrated under reduced pressure and dried under high vacuum to yield 100 as the HCl salt.

### Example 2. Synthesis of (+)-3-Isopropoxy-17-methyl-(9α,13α,14α)-morphinan (102). Compound 102 can be prepared as outlined in Scheme 5 below. Details of a synthesis are set forth below.

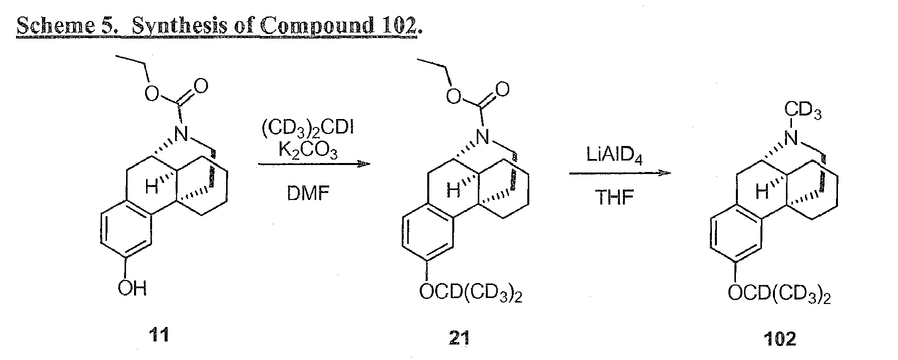

### Synthesis of (+)-3-isopropoxy-17-ethoxycarbonyl-(9α,13α,14α)-morphinan (21).

To a solution of alcohol **11** (1.50 g, 4.8 mmol; produced according to Example 1) in DMF (25 mL), is added K₂CO₃ (2.00 g, 14.5 mmol, 3.05 eq) and 2-iodopropane (0.71 mL, 7.1 mmol, 1.50 eq) with stirring. The reaction mixture is stirred overnight at room temperature under an atomosphere of N₂, then quenched by the addition of H₂O, and extracted with Et₂O (3 x 30 mL). The combined organics are dried over Na₂SO₄, filtered and concentrated *in vacuo.* Purification via automated flash column chromatography (0-40% EtOAc/hexanes) affords intermediate **21.**

**Synthesis of (+)-3-isopropoxy-17-methyl-(9α,13α,14α)-morphinan (102).** To a slurry of LiAlH₄ (0.340 g, 8.1 mmol, 4.0 eq) in THF (10 mL) stirring at -78 °C is added a solution of the carbamate 21 (0.739 g, 2.0 mmol) in THF (5 mL). The reaction mixture is stirred overnight at rt, then is quenched by the addition of magnesium sulfate heptahydrate until cessation of gas evolution. The mixture is filtered, the filtrate concentrated *in vacuo* and the resultant material is dissolved in CH₃OH. The resulting solution is acidified to pH 4 with fumaric acid resulting in salt precipitation. The mixture is stirred for 5 min, and Et₂O is added to bring remaining salt out of solution. The salt is isolated by filtration and dried to yield 102 as the fumaric acid salt

### Example 3. Evaluation of Metabolic Stability in CYP2D6 SUPERSOMES^{TM}.

Human CYP2D6 SUPERSOMES™ can be purchased from GenTest (Woburn, MA, USA). 7.5 mM stock solutions of test compounds are prepared in DMSO. The 7.5 mM stock solutions are diluted to 50 µM in acetonitrile (ACN). The 1000 pmol/mL CYP2D6 supersomes are diluted to 62.5 pmol/mL in 0.1 M potassium phosphate buffer, pH 7.4, containing 3 mM MgCl₂. The diluted SUPERSOMES™ are added to wells of a 96-well deep-well polypropylene plate in triplicate. 10 µL of the 50 µM test compound are added to the supersomes and the mixture is pre-warmed for 10 minutes. Reactions are initiated by addition of pre-warmed NADPH solution. The final reaction volume is 0.5 mL and contained 50 pmol/mL CYP2D6 SUPERSOMES™, 1 µM test compound, and 2 mM NADPH in 0.1 M potassium phosphate buffer, pH 7.4, and 3 mM MgCl₂. The reaction mixtures are incubated at 37°C and 50 µL aliquots are removed at 0, 5, 10, 20, and 30 minutes and added to shallow-well 96-well plates which contained 50 µL of ice-cold ACN with internal standard to stop the reactions. The plates are stored at 4°C for 20 minutes after which 100 µL of water is added to the wells of the plate before centrifugation to pellet precipitated proteins. Supernatants are transferred to another 96-well plate and analyzed for amounts of parent remaining by LC-MS/MS using an Applied Bio-systems API 4000 mass spectrometer.

The *in vitro* half-life (t_{1/2}) for each of the test compounds is calculated from the slopes of the linear regression of % parent remaining (ln) vs incubation time relationship: in vitro t_{½} = 0.693/k, where k = -[slope of linear regression of % parent remaining(ln) vs incubation time]. Data analysis is performed using Microsoft Excel Software..

Without further description, it is believed that one of ordinary skill in the art can, using the preceding description and the illustrative examples, make and utilize the compounds provided herein and practice the claimed methods. It should be understood that the foregoing discussion and examples merely present a detailed description of certain embodiments. It will be apparent to those of ordinary skill in the art that various modifications and equivalents can be made without departing from the spirit and scope of the disclosure. All the patents, journal articles and other documents discussed or cited above are herein incorporated by reference.

## Claims

1. A combination of:
(a) a therapeutically effective amount of a selective serotonin reuptake inhibitor; or a pharmaceutically acceptable salt thereof; and
(b) a therapeutically effective amount of a compound selected from the group consisting of a compound of Formula I: 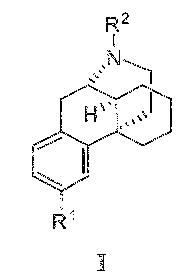 or a pharmaceutically acceptable salt thereof, wherein:
R¹ is selected from -O-(C₂-C₄)alkyl and -(C₁-C₄)alkyl; and
R² is -CH₃; and
a compound of Formula II: 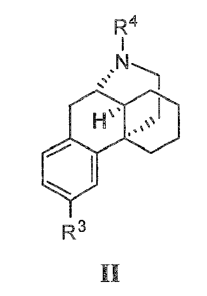 or a pharmaceutically acceptable salt thereof, wherein:
R³ is -OCH₃; and
_{R}⁴ is -CH₃;
for use in treating chronic or intractable pain.

2. The combination for use of claim 1, wherein said compound (b) is a compound of Formula **I**.

3. The combination for use of claim 2, wherein R¹ is -O-CH₂CH₃, -O-CH(CH₃)₂, -CH₃, -CH₂CH₃, or -CH₂CH(CH₃)₂.

4. The combination for use of claim 1, wherein said compound (b) is a compound of Formula **II**.

5. The combination for use of claim 1, wherein said co-agent is an inhibitor of a cytochrome p450 2D6 enzyme.

6. The combination for use of any one of claims 1-5, wherein said selective serotonin reuptake inhibitor is selected from the group consisting of fluoxetine, norfluoxetine, citalopram, dapoxetine, escitalopram, fluvoxamine, paroxetine, and sertraline, or pharmaceutically acceptable salts thereof.

7. The combination for use of claim 6, wherein said selective serotonin reuptake inhibitor is selected from the group consisting of citalopram, norfluoxetine, dapoxetine, escitalopram, fluvoxamine, paroxetine, and sertraline, or pharmaceutically acceptable salts thereof.

8. The combination for use of claim 7, wherein said selective serotonin reuptake inhibitor is paroxetine, or a pharmaceutically acceptable salt thereof.

9. The combination for use of claim 1, wherein:
said selective serotonin reuptake inhibitor is selected from the group consisting of citalopram, fluvoxamine, norfluoxetine, fluoxetine, paroxetine and sertraline, or pharmaceutically acceptable salts thereof, and
said compound of formula I is selected from the group consisting of: 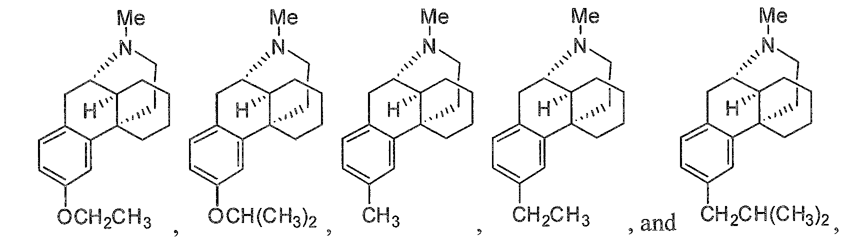 or pharmaceutically acceptable salts thereof.

10. The combination for use of claim 1, wherein:
said selective serotonin reuptake inhibitor is selected from the group consisting of citalopram, fluvoxamine, norfluoxetine, fluoxetine, paroxetine and sertraline, or pharmaceutically acceptable salts thereof, and
said compound of formula II is: 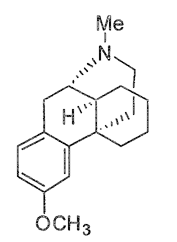 or a pharmaceutically acceptable salt thereof.

11. The combination for use of claim 9 or claim 10, wherein said selective serotonin reuptake inhibitor is selected from the group consisting of citalopram, fluvoxamine, paroxetine and sertraline or pharmaceutically acceptable salts thereof.

12. The combination for use of claim 9 or claim 10, wherein said selective serotonin reuptake inhibitor is paroxetine, or a pharmaceutically acceptable salt thereof.

13. The combination for use of any one of claims 1-12, wherein said chronic or intractable pain is a neuropathic pain.

14. The combination for use of any one of claims 1-12, wherein said chronic or intractable pain is diabetic neuropathic pain.
